# EUROPEAN PATENT SPECIFICATION

(11) **EP 4 416 205 B1**
(45) Date of publication and mention of the grant of the patent: **10.12.2025**
(21) Application number: 22802933.6
(22) Date of filing: 13.10.2022
(51) Int. Cl.: C08G 63/685, A01N 43/00, C09D 4/06, C09D 167/07, A61K 38/00

(54) **ZWITTERIONIC FUNCTIONALIZED POLY(BETA-AMINOESTER) POLYMERS AND USES THEREOF**
ZWITTERIONISCHE FUNKTIONALISIERTE POLY(BETA-AMINOESTER)-POLYMERE UND DEREN VERWENDUNGEN
POLYMÈRES POLY(BÊTA-AMINOESTER) FONCTIONNALISÉS ZWITTÉRIONIQUES ET LEURS UTILISATIONS

(30) Priority: 14.10.2021 EP 21382923
(43) Date of publication of application: 21.08.2024
(73) Proprietor: Institut Químic de Sarrià CETS Fundació Privada, 08017 Barcelona (ES)
(72) Inventor: BORRÓS GÓMEZ, Salvador, 08017 BARCELONA (ES); FORNAGUERA PUIGVERT, Cristina, 08017 BARCELONA (ES); GARCIA FERNANDEZ, Coral, 08017 BARCELONA (ES)
(74) Representative: ZBM Patents - Zea, Barlocci & Markvardsen
(86) International application number: PCT/EP2022/078472
(87) International publication number: WO 2023/062114

(56) References cited:
- US-A1- 2018 000 968
- US-A1- 2018 250 410
- US-A1- 2020 147 237
- BRUGADA-VILÀ PAU ET AL: "Oligopeptide-modified poly(beta-amino ester)s-coated AdNuPARmE1A: Boosting the efficacy of intravenously administered therapeutic adenoviruses", THERANOSTICS, vol. 10, no. 6, 3 February 2020 (2020-02-03), AU, pages 2744 - 2758, XP055881758, ISSN: 1838-7640, DOI: 10.7150/thno.40902
- RIERA ROGER ET AL: "Tracking the DNA complexation state of pBAE polyplexes in cells with super resolution microscopy", vol. 11, no. 38, 17 September 2019 (2019-09-17), United Kingdom, pages 17869 - 17877, XP055904133, ISSN: 2040-3364, Retrieved from the Internet <URL:https://pubs.rsc.org/en/content/articlepdf/2019/nr/c9nr02858g> [retrieved on 20220322], DOI: 10.1039/C9NR02858G
- DOSTA P. ET AL: "Stable and efficient generation of poly([beta]-amino ester)s for RNAi delivery", MOLECULAR SYSTEMS DESIGN & ENGINEERING, vol. 3, no. 4, 17 May 2018 (2018-05-17), pages 677 - 689, XP055904349, Retrieved from the Internet <URL:https://pubs.rsc.org/en/content/articlepdf/2018/me/c8me00006a> [retrieved on 20220323], DOI: 10.1039/C8ME00006A

## Description

### Technical Field

The invention relates to polymers suitable for use in the delivery of active agents. The invention also pertains to nanoparticles comprising these polymers, compositions comprising said nanoparticles and methods for their production.

### Background Art

The lack of safe and efficient vectors to deliver active ingredients such as polynucleotides (DNA, RNA and the like) remains the principal handicap for the success of gene therapy. The majority of protocols for polynucleotide delivery employ viral vectors, which are highly efficient delivery systems. However, viral vectors have certain disadvantages, including safety risk, limited capacity to carry polynucleotides and high cost of large-scale production. Non-viral vectors offer potential advantages, including high packing capacity, ease of production, low toxicity and immunogenicity, but are less efficient than viral vectors.

Biodegradable poly(beta-aminoester)s (pBAE)s have been described as potential non-viral polynucleotide delivery vectors capable of condensing both DNA and RNA into discrete nanometric particles.

A continuing need exists for improved non-toxic, biodegradable, biocompatible polymers that can be used to transfect polynucleotides efficiently and that can be prepared economically. Such polymers would be useful in the packaging and delivery of DNA and RNA in gene therapy and for the packaging and delivery of other diagnostic, therapeutic and prophylactic agents.

In addition to the above, it is well-known that delivery systems such as nanoparticles (NPs), when are exposed to biological fluids, rapidly adsorb proteins. These form the so-called "hard" and "soft" corona according to their binding strength and exchange rates at the NP surface. The composition of this protein corona depends on many parameters, including particle size, surface chemistry (hydrophilicity, surface charge, etc.), environmental conditions (time, temperature, etc.) and the nature and composition of biological fluids. However, in many *in vivo* applications, the protein corona dictates the fate of nanocarriers and induces fast recognition by the immune system. It, therefore, reduces the circulation lifetime in the blood stream, as well as the targeting efficiency, and influences cellular uptake, biodistribution and toxicity.

US2018000968A1 discloses nanoparticles that are suitable for use in delivery of active agents comprising PBAES and additives that are sugars or sugar derivatives. Brugada-Vilà, P. et al., Theranostics, 2020, 10(6), p. 2744 discloses oligopeptide-modified pBAEs for use as NP in cancer therapy. Riera, R. et al., Nanoscale, 2019, 11(38), p. 17869 relates to tracking the DNA complexation state of pBAE polyplexes in cells.

In view of the above, there is the need of polymers with improved properties, and which overcome the above identified problems.

### Summary of Invention

The present invention provides novel zwitterionic-modified pBAEs with improved properties, making them useful in a variety of medical applications, including drug delivery, as defined by the appended claims.

As it is shown below, the modified pBAE polymers of the invention, hereinafter also referred as "OM-PZ-pBAE", which are characterized by being covalently modified with zwitterionic polymeric side-chains, provide improved anti-fouling properties. In this regard, the inventors prepared nanoparticles with the polymer of the invention and performed a test simulating an *in vivo* environment. Table 9 below shows that the polymer of the invention (Example 6.3) can remarkably reduce the adhesion of proteins to particle's surface. Particularly, it can be seen that the polymers of the invention give rise to an about 7-fold reduction in the protein corona formation when compared with the same polymer pBAE lacking the zwitterionic polymer (comparative example 7), besides the cationic charges of the pBAE backbone.

Not only that, but also, the data provided below show that the covalent binding of the zwitterionic polymer to the pBAE backbone is the responsible for such surprising improvement. In this regard, it is provided comparative Example 8, which differs from the polymers of the invention uniquely in the nature of the interaction between the zwitterionic polymer and the pBAE backbone: covalent (invention) vs electrostatic (comparative Example 8). As it can be concluded from Table 9 below, the behaviour of the pBAE comprising the electrostatically bound zwitterionic polymer is substantially the same as the one of the pBAE with no zwitterionic polymer in the *in vivo* test, being substantially less efficient in preventing the adhesion of proteins on nanoparticle's surface than the pBAE of the invention, which is covalently modified with zwitterionic polymers. The latter means that it is not only necessary the presence of a zwitterionic polymer, but the most important, that the zwitterionic polymer has to be covalently linked (i.e. grafted) to the pBAE backbone, as it is the case of the polymers of the invention, to achieve such remarkable improvement in the anti-fouling properties.

The excellent anti-fouling properties provided by the polymers of the invention, due to the grafting of the zwitterionic polymer, confer, not only stability to the nanoparticles, but also, protection from the adherence of protein to the optional targeting moieties functionalizing the nanoparticles of the invention. Therefore, the therapeutic efficiency can also be improved.

Advantageously, the inventors also found that the covalent binding of the zwitterionic polymer to the pBAE backbone did not negatively affect to the encapsulation efficiency, neither to the suitable size of the nanoparticles to be used as a delivery vehicle.

In particular, it was found that the nanoparticles of the invention had low average size, remarkably lower than 1000 nm, as it is shown in Tables 7-8 below. The above is of relevance because a size below 1000 nm guarantees that the nanoparticles can provide the biologically effect: (a) it is minimized the formation of aggregates, and (b) it is facilitated cell internalization. In addition, small sizes guarantee an appropriate stability when formulated in the form of a pharmaceutical composition and can be easily reproduced.

The administration of nanocarriers or vehicles is usually systemic and performed through parenteral routes. Therefore, nanoparticles get in contact with blood very frequently. As it is shown below, the inventors also determined the compatibility of the polymers of the invention with red blood cells (i.e. erythrocytes), which is a must before driving *in vivo* studies: if the nanoparticle solution is hypoosmotic, erythrocytes will explode; while hyperosmotic solution produce a shrinkage of erythrocytes.

Surprisingly, it was also found that the polymers of the invention not only extended the life of the nanoparticle, but also, they provided a lower % of haemolysis, even at high doses of active ingredient. This behaviour was not found when the zwitterionic polymer was electrostatically interacting with the pBAE polymer (Table 11, below).

This means that the covalent grafting of zwitterionic chains into the pBAE backbone confers improved properties to the resulting nanoparticles, which are based on a remarkably stability but also on a remarkably higher security for their use in therapy, without negatively affecting to the encapsulating efficiency or ability to diffuse. Altogether this means a great advance in the field of pharmaceuticals, especially in the development of suitable pharmaceutical carriers which are highly-efficient encapsulating systems, highly-stable due to a remarkable anti-fouling effect, and safer due to the remarkable low cytotoxicity or significant absence of any haemolytic effect.

Thus, in a first aspect the present invention provides a poly(beta-aminoester) polymer of formula (I) or a pharmaceutically salt thereof: 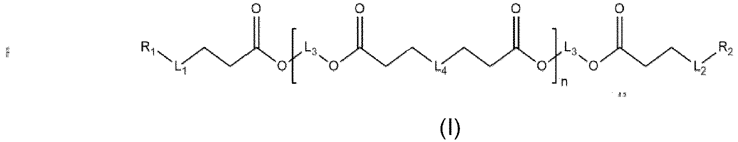 wherein
L₃ is independently selected from the group consisting of alkylene, alkenylene, heteroalkylene, heteroalkenylene, arylene, and heteroarylene; or, alternatively
at least one of L₃ is
   wherein T₁ is 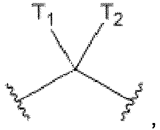 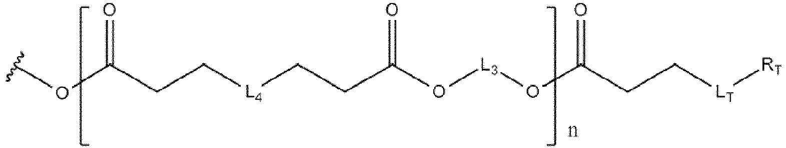
   and T₂ is selected from H, alkyl, and 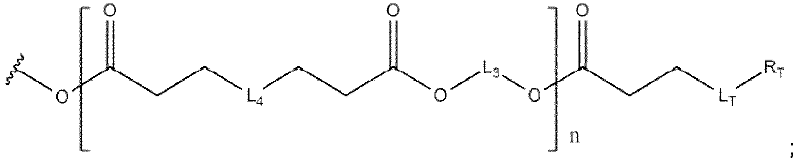
   wherein L_{T} is independently selected from the group consisting of: 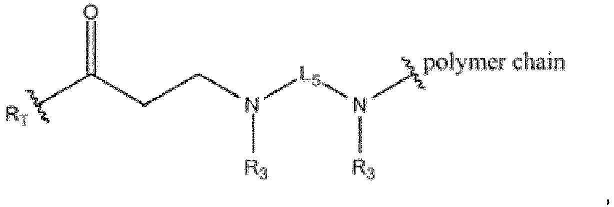
   O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl,
and the remaining L₃ groups are independently selected from the group consisting of alkylene, alkenylene, heteroalkylene, heteroalkenylene, arylene, and heteroarylene;
L₄ is independently selected from the group consisting of formula (i) and (ii) 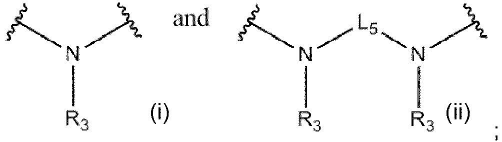
L₅ is independently selected from the group consisting of alkylene, alkenylene, heteroalkylene, heteroalkenylene, arylene, and heteroarylene;
each R₃ is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), a moiety having an amine group (-NH₂), and a moiety having a zwitterionic polymer, wherein:
   - said polyalkylene glycol is either bound directly to the nitrogen atom to which R₃ is attached or bound to the nitrogen atom to which R₃ is attached via a linker moiety, wherein said linker moiety is an alkylene, cycloalkylene, alkenylene, cycloalkenylene, heteroalkylene, heterocycloalkylene, arylene or heteroarylene group;
   - the moiety having a hydroxyl group (-OH) is selected from the group consisting of -(CH₂)ₚOH, and -(CH₂)₂(OCH₂CH₂)_{q}OH;
   - the moiety having an amino group (-NH₂) is selected from the group consisting of -(CH₂)ₚNH₂, and -(CH₂)₂(OCH₂CH₂)_{q}NH₂; and
   - the moiety having a zwitterionic polymer is one of formula (II) 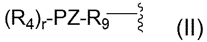
      wherein R₄ represents R'₄-C(=S)-S-; R'₄ represents an aryl, heteroaryl, alkyl, -SR₅, -NR₆R₇ or -ORₐ; R₅ represents aryl, heteroaryl or alkyl; R₆ and R₇ are the same or different and represent hydrogen, halogen, alkyl, cycloalkyl, alkenyl, cycloalkenyl, heteroalkyl, heterocycloalkyl, acyl, aryl, and heteroaryl; R₈ represents hydrogen, alkyl, cycloalkyl, alkenyl, cycloalkenyl, heteroalkyl, heterocycloalkyl, acyl, aryl, and heteroaryl; and R₉ is a linker which binds the PZ to the -N- of the substituent of formula (i) or (ii);
      PZ represents a zwitterionic polymer comprising one or more zwitterionic monomers;
      provided that at least one of the R₃ is a moiety having a zwitterionic polymer as defined above;
n is an integer from 5 to 1000, p is an integer from 1 to 20, q is an integer from 1 to 10, and r is an integer from 0 to 1;
each L₁ and L₂ is independently selected from the group consisting of: 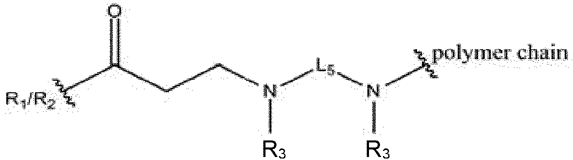
O, S, NRₓ, and a bond; wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl; and R₃ and L₅ being as defined above; and
R₁, R₂ and R_{T} are independently selected from an oligopeptide and R_{y}, R_{y} being selected from the group consisting of hydrogen, halogen, alkyl, cycloalkyl, alkenyl, cycloalkenyl, heteroalkyl, heterocycloalkyl, acyl, aryl, and heteroaryl; and
provided that at least one of R₁, R₂ and R_{T} is an oligopeptide.

In a second aspect the present disclosure provides a process for preparing a polymer as defined in the first aspect of the invention, the process comprising the step of performing a reversible addition-fragmentation chain transfer (RAFT) polymerization of a polymer of formula (Ibis) the process comprising the step of performing the RAFT polymerization of a polymer of formula (Ibis) 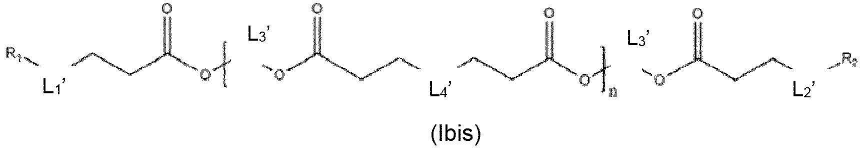
with one or more zwitterionic monomers, in the presence of a radical initiator,
wherein
L₃' is independently selected from the group consisting of alkylene, alkenylene, heteroalkylene, heteroalkenylene, arylene, and heteroarylene; or, alternatively at least one of L₃' is 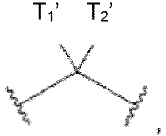 **wherein**
   T₁' is 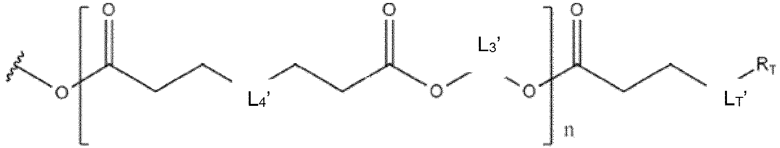
   and T₂' is selected from H, alkyl, and 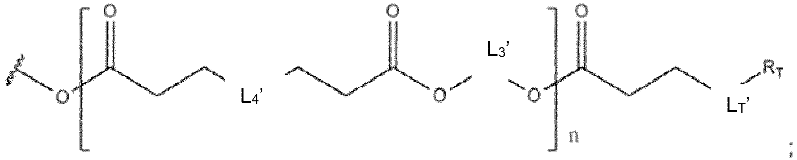
   wherein L_{T}' is independently selected from the group consisting of: 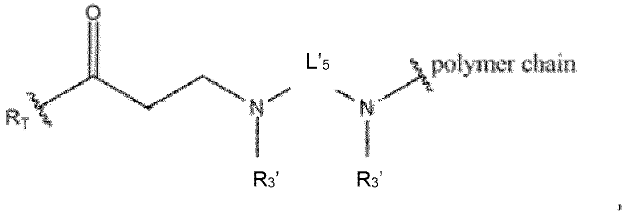
   O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl,
and the remaining L'₃ groups are independently selected at each occurrence from the group consisting of alkylene, alkenylene, heteroalkylene, heteroalkenylene, arylene, and heteroarylene;
L'₄ is independently selected from the group consisting of 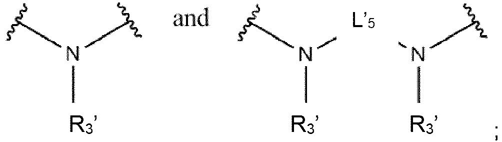
L'₅ is independently selected from the group consisting of alkylene, alkenylene, heteroalkylene, heteroalkenylene, arylene, and heteroarylene;
each R₃' is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), a moiety having an amine group (-NH₂), and a moiety having a chain transfer agent (CTA); provided that at least one or more of the R₃' are moieties having a CTA,
wherein:
   - said polyalkylene glycol is either bound directly to the nitrogen atom to which R₃ is attached or bound to the nitrogen atom to which R₃ is attached via a linker moiety, wherein said linker moiety is an alkylene, cycloalkylene, alkenylene, cycloalkenylene, heteroalkylene, heterocycloalkylene, arylene or heteroarylene group;
   - the moiety having a hydroxyl group (-OH) is selected from the group consisting of -(CH₂)ₚOH, and -(CH₂)₂(OCH₂CH₂)_{q}OH;
   - the moiety having an amino group (-NH₂) is selected from the group consisting of -(CH₂)ₚNH₂, -(CH₂)₂(OCH₂CH₂)_{q}NH₂;
   - the moiety having a CTA corresponds to a thio-based compound of formula (IV) 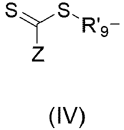
wherein
Z represents an aryl, heteroaryl, alkyl, -SR'₅, -NR'₆R'₇ or -OR'₃;
R'₅ represents aryl, heteroaryl or alkyl;
R'₆ and R'₇ are the same or different and represent hydrogen, halogen, alkyl, cycloalkyl, alkenyl, cycloalkenyl, heteroalkyl, heterocycloalkyl, acyl, aryl, and heteroaryl;
R'₈ represents hydrogen, alkyl, cycloalkyl, alkenyl, cycloalkenyl, heteroalkyl, heterocycloalkyl, acyl, aryl, and heteroaryl; and
R'₉ is a linker which binds the -S- of the thio-based compound to the -N- of the group of formula (i) or (ii);
n is an integer from 5 to 1000, p is an integer from 1 to 20, q is an integer from 1 to 10, each L₁ and L₂ is independently selected from the group consisting of: 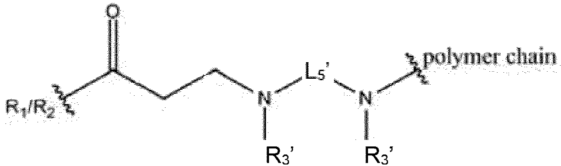
O, S, NRₓ, and a bond; wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl; and R₃' and L'₅ being as defined above; and
R₁, R₂ and R_{T} are independently selected from an oligopeptide and R_{y}, R_{y} being selected from the group consisting of hydrogen, halogen, alkyl, cycloalkyl, alkenyl, cycloalkenyl, heteroalkyl, heterocycloalkyl, acyl, aryl, and heteroaryl; and
provided that at least one of R₁, R₂ and R_{T} is an oligopeptide.

In a third aspect the present disclosure provides a polymer of formula (I) obtainable by the process of the second aspect of the invention.

In a fourth aspect the present invention provides a nanoparticle, comprising one or more polymers of formula (I) as defined in the first and/or third aspects of the invention.

In a fifth aspect the present invention provides a pharmaceutical composition comprising (a) a therapeutically effective amount of the nanoparticle, as defined in the fourth aspect of the invention, comprising an active ingredient, and (b) one or more pharmaceutically acceptable excipients or carriers.

The surprising properties conferred by the polymers of the invention make them also suitable for use as a coating of any other delivery system to improve the stability (anti-fouling effect) as well-as as its safety.

In a sixth aspect the present invention provides the use of the polymer of formula (I) as defined in the first or third aspect of the invention, alone or in combination with one or more polymers of formula (Iter) as defined below, as a coating, particularly as anti-fouling coating.

In a seventh aspect the present invention provides a delivery system comprising an external coating comprising the polymer (I) as defined in the first or third aspect of the invention and, optionally, one or more polymers of formula (Iter) as defined below.

In an eighth aspect the present invention provides the use of the polymer as defined in the first or thrid aspect of the invention, alone or in combination with one or more polymers of formula (Iter) as defined below, as a delivery carrier.

In a ninth aspect the present invention provides the use of the polymer as defined in the first or third aspect of the invention, alone or in combination with one or more polymers of formula (Iter) as defined below, as an encapsulating agent.

In a tenth aspect the present invention provides the pharmaceutical composition as defined in the fifth aspect of the invention or the nanoparticle as defined in the fourth aspect of the invention or the coated viral particle as defined in the seventh aspect of the invention for use in therapy.

In an eleventh aspect the present disclosure provides a method of encapsulating an agent in a matrix of one or more polymers of formula (I) as defined in the first or third aspect of the invention, optionally in combination with one or more polymers of formula (Iter) as defined below, the method comprising steps of:
providing an active agent as defined above;
providing the polymer (s); and
contacting the agent and the polymer under suitable conditions to form nanoparticles.

In a last aspect the present disclosure provides a method for the preparation of a coating viral particle as defined in the seventh aspect of the invention, the method comprising the step of mixing the viral particle with the polymer(s) as defined in the first aspect of the invention, optionally in combination with one or more polymers of formula (Iter) as defined below.

### Detailed description of the invention

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

It is noted that, as used in this specification and the appended claims, the singular forms "a", "an", and "the" include plural referents unless the context clearly dictates otherwise. For the purposes of the present invention, any ranges given include both the lower and the upper end-points of the range.

The present invention provides in a first aspect, pBAE polymers covalently modified with zwitterionic polymers.

In one embodiment of the first aspect of the invention, L₃ is independently selected from the group consisting of alkylene, alkenylene, heteroalkylene, heteroalkenylene, arylene, and heteroarylene; particularly L₃ represents alkylene.

In another embodiment of the first aspect of the invention, optionally in combination with any of the embodiments provided above or below, L₄ represents

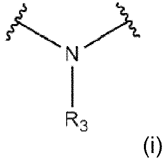

In another embodiment of the first aspect of the invention, optionally in combination with any of the embodiments provided above or below, at least a 5% of the R₃ groups in the polymer of formula (I) represents a moiety having a zwitterionic polymer of formula (II) as defined in any of the above clauses; particularly at least a 30%, 40%, 50%, 60%, 70%, 80% or 90% of the R₃ groups in the polymer of formula (I) represents a moiety having a zwitterionic polymer of formula (II) as defined in any of the above clauses; particularly at least a 40% of the R₃ groups in the polymer of formula (I) represents a moiety having a zwitterionic polymer of formula (II) as defined in any of the above clauses; particularly from 30 to 80%; particularly from 40 to 70% of the R₃ groups in the polymer of formula (I) represents a moiety having a zwitterionic polymer of formula (II) as defined in any of the above clauses.

In another embodiment of the first aspect of the invention, optionally in combination with any of the embodiments provided above or below, at least a 5% of the R₃ groups in the polymer of formula (I) represents a moiety having a zwitterionic polymer of formula (II) as defined in above and the remaining R₃ groups represent a moiety having an amino group as defined above; particularly at least a 30%, 40%, 50%, 60%, 70%, 80% or 90% of the R₃ groups in the polymer of formula (I) represents a moiety having a zwitterionic polymer of formula (II) as defined in above and the remaining R₃ groups represent a moiety having an amino group as defined above; particularly at least a 40% of the R₃ groups in the polymer of formula (I) represents a moiety having a zwitterionic polymer of formula (II) as defined above and the remaining R₃ groups represent a moiety having an amino group as defined above; particularly from 30 to 80%; particularly from 40 to 70% of the R₃ groups in the polymer of formula (I) represents a moiety having a zwitterionic polymer of formula (II) as defined above and the remaining R₃ groups represent a moiety having an amino group as defined above.

The % of R₃ groups in the polymer of formula (I) representing a moiety having a zwitterionic polymer of formula (II) is calculated dividing the number of R₃ groups meaning the moiety of formula (II) by the total number of groups R₃ forming part of the polymer of formula (I). The number of R₃ groups is determined by 1H-NMR, integrating the signal(s) of carbon atom of R₃ located more distal from pBAE backbone.

In another embodiment of the first aspect of the invention, optionally in combination with any of the embodiments provided above or below, the remaining R₃ groups forming part of the OM-PZ-pBAE, which are other than a moiety having a zwitterionic polymer, are the same or different and are selected from: -(CH₂)ₚOH, -(CH₂)₂(OCH₂CH₂)_{q}OH, -(CH₂)ₚNH₂, and -(CH₂)₂(OCH_{2C}H₂)_{q}NH₂, wherein p and q are as defined above. In one embodiment, optionally in combination with any of the embodiments provided above or below, p and q represents from 1 to 10.

In another embodiment of the first aspect of the invention, optionally in combination with any of the embodiments provided above or below the L₁ and/or L₂ linking the or each oligopeptide to the polymer is a bond.

In another embodiment of the first aspect of the invention, optionally in combination with any of the embodiments provided above or below, Ry is selected from a group consisting of hydrogen, -(CH₂) ₘNH₂, -(CH₂)mNHMe, -(CH₂)mOH, -(CH₂)mCH₃, -(CH₂) ₂ (OCH₂CH₂)mNH₂, -(CH₂)₂(OCH₂CH₂)mOH or -(CH₂)₂(OCH₂CH₂)mCH₃ wherein m is an integer from 1 to 20.

In another embodiment of the first aspect of the invention, optionally in combination with any of the embodiments provided above or below, the polymer of formula (I) is one, wherein:
R₁ and R₂ are oligopeptides as defined below, particularly negatively charged olipeptides at pH 7;
L₁ and L₂ represent a bond;
L₃ represents alkylene;
L₄ represents a substituent of formula (i), wherein:
   - at least a 5% of the R₃ groups represent a moiety having a zwitterionic polymer of formula (II) as defined above; R'₄ being selected from alkyl, aryl, -S-alkyl, and -S-aryl; R₉ representing -alkylene-C(O)-O-alkylene-, wherein the alkylene is optionally substituted; and PZ is as defined in any of the embodiments of the first aspect of the invention provided below; and
   - the remaining R₃ groups, which are other than the moiety of formula (II), are the same or different and are selected from -(CH₂)ₚOH, -(CH₂)₂(OCH₂CH₂)_{q}OH, -(CH₂)ₚNH₂, and -(CH₂)₂(OCH₂CH₂)_{q}NH₂.

The expression "L₁ represent a bond" means that the polymer of formula (I) is one wherein:

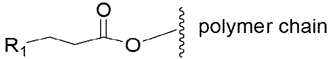

The expression "L₂ represent a bond" means that the polymer of formula (I) is one wherein:

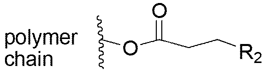

### Chemical groups

The term "halogen" (or "halo") includes fluorine, chlorine, bromine and iodine.

The term "alkyl" includes monovalent, straight or branched, saturated, acyclic hydrocarbyl groups. Alkyl is suitably C₁₋₁₀alkyl, or C₁₋₆alkyl, or C₁₋₄alkyl, such as methyl, ethyl, n-propyl, i-propyl or t-butyl groups. Alkyl may be substituted.

The term "cycloalkyl" includes monovalent, saturated, cyclic hydrocarbyl groups. Cycloalkyl is suitably C₃₋₁₀cycloalkyl, or C₃₋₆cycloalkyl such as cyclopentyl and cyclohexyl. Cycloalkyl may be substituted.

The term "alkoxy" means alkyl-O-.

The term "alkylamino" means alkyl-NH-.

The term "alkenyl" includes monovalent, straight or branched, unsaturated, acyclic hydrocarbyl groups having at least one carbon-carbon double bond and, suitably, no carbon-carbon triple bonds. Alkenyl is suitably C₂₋₁₀alkenyl, or C₂₋₆alkenyl, or C₂₋₄alkenyl. Alkenyl may be substituted.

The term "cycloalkenyl" includes monovalent, partially unsaturated, cyclic hydrocarbyl groups having at least one carbon-carbon double bond and, suitably, no carbon-carbon triple bonds. Cycloalkenyl is suitably C₃₋₁₀cycloalkenyl, or C₅₋₁₀cycloalkenyl, e.g. cyclohexenyl or benzocyclohexyl. Cycloalkenyl may be substituted.

The term "alkynyl" includes monovalent, straight or branched, unsaturated, acyclic hydrocarbyl groups having at least one carbon-carbon triple bond and, suitably, no carbon-carbon double bonds. Alkynyl is suitably C₂₋₁₀alkynyl, or C₂₋₆alkynyl, or C₂₋₄alkynyl. Alkynyl may be substituted.

The term "alkylene" includes divalent, straight or branched, saturated, acyclic hydrocarbyl groups. Alkylene is suitably C₁₋₁₀alkylene, or C₁₋₆alkylene, or C₁₋₄alkylene, such as methylene, ethylene, n-propylene, i-propylene or t-butylene groups. Alkylene may be substituted.

The term "alkenylene" includes divalent, straight or branched, unsaturated, acyclic hydrocarbyl groups having at least one carbon-carbon double bond and, suitably, no carbon-carbon triple bonds. Alkenylene is suitably C₂₋₁₀alkenylene, or C₂₋₆alkenylene, or C₂₋₄alkenylene. Alkenylene may be substituted.

The term "heteroalkyl" includes alkyl groups, for example, C₁₋₆₅alkyl groups, C₁₋₁₇alkyl groups or C₁₋₁₀alkyl groups, in which up to twenty carbon atoms, or up to ten carbon atoms, or up to two carbon atoms, or one carbon atom, are each replaced independently by O, S(O)ₜ or N, provided at least one of the alkyl carbon atoms remains. The heteroalkyl group may be C-linked or hetero-linked, i.e. it may be linked to the remainder of the molecule through a carbon atom or through O, S(O)ₜ or N, wherein t is defined below. Heteroalkyl may be substituted.

The term "heterocycloalkyl" includes cycloalkyl groups in which up to ten carbon atoms, or up to two carbon atoms, or one carbon atom, are each replaced independently by O, S(O)ₜ or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, i.e. it may be linked to the remainder of the molecule through a carbon atom or through a nitrogen atom. Heterocycloalkyl may be substituted.

The term "heteroalkenyl" includes alkenyl groups, for example, C₁₋₆₅alkenyl groups, C₁₋₁₇alkenyl groups or C₁₋₁₀alkenyl groups, in which up to twenty carbon atoms, or up to ten carbon atoms, or up to two carbon atoms, or one carbon atom, are each replaced independently by O, S(O)ₜ or N, provided at least one of the alkenyl carbon atoms remains. The heteroalkenyl group may be C-linked or hetero-linked, i.e. it may be linked to the remainder of the molecule through a carbon atom or through O, S(O)ₜ or N.

Heteralkenyl may be substituted.

The term "heterocycloalkenyl" includes cycloalkenyl groups in which up to three carbon atoms, or up to two carbon atoms, or one carbon atom, are each replaced independently by O, S(O)ₜ or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, i.e. it may be linked to the remainder of the molecule through a carbon atom or through a nitrogen atom. Heterocycloalkenyl may be substituted.

The term "heteroalkynyl" includes alkynyl groups, for example, C₁₋₆₅alkynyl groups, C₁₋₁₇alkynyl groups or C₁₋₁₀alkynyl groups, in which up to twenty carbon atoms, or in which up to ten carbon atoms, or up to two carbon atoms, or one carbon atom, are each replaced independently by O, S(O)ₜ or N, provided at least one of the alkynyl carbon atoms remains. The heteroalkynyl group may be C-linked or hetero-linked, i.e. it may be linked to the remainder of the molecule through a carbon atom or through O, S(O)ₜ or N. Heteroalkynyl may be substituted.

The term "heteroalkylene" includes alkylene groups, for example, C₁₋₆₅alkylene groups, C₁₋₁₇alkylene groups or C₁₋₁₀alkylene groups, in which up to twenty carbon atoms, or in which up to ten carbon atoms, or up to two carbon atoms, or one carbon atom, are each replaced independently by O, S(O)ₜ or N, provided at least one of the alkylene carbon atoms remains. Heteroalkynylene may be substituted.

The term "heteroalkenylene" includes alkenylene groups, for example, C₁₋₆₅alkenylene groups, C₁₋₁₇alkenylene groups or C₁₋₁₀alkenylene groups, in which up to twenty carbon atoms, or in which up to ten carbon atoms, or up to two carbon atoms, or one carbon atom, are each replaced independently by O, S(O)ₜ or N, provided at least one of the alkenylene carbon atoms remains. Heteroalkenylene may be substituted.

The term "aryl" includes monovalent, aromatic, cyclic hydrocarbyl groups, such as phenyl or naphthyl (e.g. 1-naphthyl or 2-naphthyl). In general, the aryl groups may be monocyclic or polycyclic fused ring aromatic groups. Preferred aryl are C₆-C₁₄aryl. Aryl may be substituted.

Other examples of aryl groups are monovalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

The term "arylalkyl" means alkyl substituted with an aryl group, e.g. benzyl.

The term "arylene" means a divalent, aromatic, cyclic hydrocarbyl groups, such as phenylene or naphthylene (e.g. 1-naphthylene or 2-naphthylene). In general, the arylene groups may be monocyclic or polycyclic fused ring aromatic groups. Preferred arylenes are C₆-C₁₄arylene. Arylene may be substituted.

The term "heteroaryl" includes aryl groups in which one or more carbon atoms are each replaced by heteroatoms independently selected from O, S, N, and NR^{N}, where R^{N} is defined below (and in one embodiment is H or alkyl (e.g. C₁₋₆alkyl)). Heteroaryl may be substituted.

In general, the heteroaryl groups may be monocyclic or polycyclic (e.g. bicyclic) fused ring heteroaromatic groups. Typically, heteroaryl groups contain 5-14 ring members (preferably 5-10 members) wherein 1, 2, 3 or 4 ring members are independently selected from O, S, N, and NR^{N}. A heteroaryl group is suitably a 5, 6, 9 or 10 membered, *e.g.* 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members wherein 1, 2, 3 or 4 ring members are independently selected from O, S, N, and NR^{N}.

5-Membered monocyclic heteroaryl groups may contain 1 ring member which is an -NR^{N}-group, an -O- atom or an -S- atom and, optionally, 1-3 ring members (e.g. 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

6-Membered monocyclic heteroaryl groups may contain 1 or 2 ring members which are =N- atoms (where the remainder of the 6 ring members are carbon atoms).

Bicyclic heteroaromatic groups include fused-ring heteroaromatic groups containing 9-14 ring members wherein 1, 2, 3, 4 or more ring members are independently selected from O, S, N, and NR^{N}.

9-Membered bicyclic heteroaryl groups may contain 1 ring member which is an -NR^{N}-group, an-O- atom or an -S- atom and, optionally, 1-3 ring members (e.g. 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

10-Membered bicyclic heteroaryl groups may contain 1-3 ring members which are =N-atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylene" means divalent aryl groups in which one or more carbon atoms are each replaced by heteroatoms independently selected from O, S, N, and NR^{N}, where R^{N} is defined below (and in one embodiment is H or alkyl (e.g. C₁₋₆alkyl)). Heteroarylene may be substituted.

The term "heteroarylalkyl" means alkyl substituted with a heteroaryl group.

Examples of acyl groups include alkyl-C(=O)-, cycloalkyl-C(=O)-, alkenyl-C(=O)-, cycloalkenyl-C(=O)-, heteroalkyl-C(=O)-, heterocycloalkyl-C(=O)-, aryl-C(=O)- or heteroaryl-C(=O)-, in particular, alkyl-C(=O)- and aryl-C(=O)-.

Unless indicated explicitly otherwise, where combinations of groups are referred to herein as one moiety, e.g. arylalkyl, the last mentioned group contains the atom by which the moiety is attached to the rest of the molecule.

Where reference is made to a carbon atom of an alkyl group or other group being replaced by O, S(O)ₜ or N, what is intended is that: 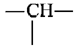 is replaced by 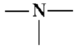
-CH= is replaced by -N=;
=C-H is replaced by ≡N; or
-CH₂- is replaced by -O-, -S(O)ₜ- or -NR^{N}-.

By way of clarification, in relation to the above mentioned heteroatom containing groups (such as heteroalkyl etc.), where a numerical of carbon atoms is given, for instance C₃₋₆heteroalkyl, what is intended is a group based on C₃₋₆alkyl in which one of more of the 3-6 chain carbon atoms is replaced by O, S(O)ₜ or N. Accordingly, a C₃₋₆heteroalkyl group, for example, will contain less than 3-6 chain carbon atoms.

Where mentioned above, R^{N} is H, alkyl, cycloalkyl, aryl, heteroaryl, -C(O)-alkyl, -C(O)-aryl, -C(O)-heteroaryl, -S(O)ₜ-alkyl, -S(O)ₜ-aryl or -S(O)ₜ-heteroaryl. R^{N} may, in particular, be H, alkyl (e.g. C₁₋₆alkyl) or cycloalkyl (e.g. C₃₋₆cycloalkyl).

Where mentioned above, t is independently 0, 1 or 2, for example 2. Typically, t is 0.

Where a group has at least 2 positions which may be substituted, the group may be substituted by both ends of an alkylene or heteroalkylene chain to form a cyclic moiety.

Optionally substituted groups (e.g. alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene, aryl, arylalkyl, arylheteroalkyl, heteroaryl, heteroarylalkyl or heteroarylheteroalkyl groups *etc*.) may be substituted or unsubstituted, or may be unsubstituted. Typically, substitution involves the notional replacement of a hydrogen atom with a substituent group, or two hydrogen atoms in the case of substitution by =O.

Where substituted, there will generally be 1 to 3 substituents, or 1 or 2 substituents, or 1 substituent.

The optional substituent(s) is/are independently halogen, trihalomethyl, trihaloethyl,-OH, - NH₂, -NO₂, -CN, -N⁺(C₁₋₆alkyl)₂O-, -CO₂H, -CO₂C₁₋₆alkyl, -SO₃H, -SOC₁₋₆alkyl, -SO₂C_{5. 6}alkyl, -SO₃C₁₋₆alkyl, -OC(=O)OC₁₋₆alkyl, -C(=O)H, -C(=O)C₁₋₆alkyl, -OC(=O)C₁₋₆alky), =O, -NH(C₁₋₆alkyl), -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, - C(=S)N(C_{I-6}alkyl)₂, -N(C₁₋₆alkyl)C(=S)C₁₋₆alkyl, -SO₂N(C₁₋₆alkyl)₂, -N(C₁₋₆alkyl)SO₂C₁₋₆alkyl, -N(C₁₋₆alkyl)C(=S)N(C₁₋₆alkyl)₂, -N(C₁₋₆alkyl)SO₂N(C₁₋₆alkyl)₂, -C₁₋₆alkyl, -C₁₋₆heteroalkyl, - C₃₋₆cycloalkyl, -C₃₋₆heterocycloalkyl, -C₂₋₆alkenyl, -C₂₋₆heteroalk enyl, -C₃₋₆cycloalkenyl, - C₃₋₆heterocycloalkenyl, -C₂₋₆alkynyl, -C₂₋₆heteroalkynyl, -Z^{u}-C₁₋₆alkyl,-Z^{u}-C₃₋₆cycloalkyl, - Z^{U}-C₂₋₆alkenyl, -Z^{u}-C₃₋₆cycloalkenyl or -Z^{u}-C₂₋₆alkynyl, wherein Z^{u} is independently O, S, NH or N(C₁₋₆alkyl).

In another embodiment, the optional substituent(s) is/are independently halogen, trihalomethyl, trihaloethyl, -NO₂, -CN, -N⁺(C₁₋₆alkyl)₂O-, -CO₂H, -SO₃H, -SOC₁₋₆alkyl, - SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or-z^{u}-C₃₋₆cycloalkyl, wherein Z^{u} is defined above.

In another embodiment, the optional substituent(s) is/are independently halogen, trihalomethyl, -NO₂, -CN, -CO₂H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or-Z^{u}-C₃₋₆cycloalkyl, wherein Z^{u} is defined above.

In another embodiment, the optional substituent(s) is/are independently halogen, -NO₂, - CN, -CO₂H, =O, -N(C₁₋₆alkyl)₂, -C₁₋₆alkyl, -C₃₋₆cycloalkyl or -C₃₋₆heterocycloalkyl.

In another embodiment, the optional substituent(s) is/are independently halogen, -OH, NH₂, NH(C₁₋₆alkyl), -N(C₁₋₆alkyl)₂, -C₁₋₆alkyl, -C₃₋₆cycloalkyl or -C₃₋₆heterocycloalkyl.

The term "polyalkylene glycol" (PAG) refers to compounds having the general formula H-[O-C_{y}H_{2y}]ₓ-OH, such as H-[O-CH₂-CH_{2]x}-OH (polyethylene glycol or PEG) and H-[O-CH(CH₃)-CH₂]ₓ-OH (polypropylene glycol). When found in a compound of the invention the PAG is bound by the bond between a carbon atom and one of the terminal hydroxyl groups e.g. in the case of PEG the substituent would be H-[O-CH₂-CH₂]ₓ-. The polyalkylene glycols used in the compounds of the invention, unless otherwise defined, may have a molecular weight of from 500 to 20,000 g/mol, preferably from 1,000 to 10,000 g/mol, more preferably from 2,000 to 5,000 g/mol, more preferably from 2,000 to 3,500 g/mol.

As used herein, the term "polymer of Formula I", "polymer of Formula Ibis", or "polymer of Formula Iter" include pharmaceutically acceptable derivatives thereof and polymorphs, isomers and isotopically labelled variants thereof.

The term "pharmaceutically acceptable derivative" includes any pharmaceutically acceptable salt, solvate, hydrate or prodrug of a polymer of formula (I), (Ibis), (Iter) or (V). The pharmaceutically acceptable derivatives suitably refers to pharmaceutically acceptable salts, solvates or hydrates of a polymer of formula (I), (Ibis), (Iter) or (V).

As used herein, the term "pharmaceutical acceptable salt" refers to those salts which are, within the scope of sound medical judgment, suitable for use in contact with the tissues of humans and lower animals without undue toxicity, irritation, allergic response, and are commensurate with a reasonable benefit/risk ratio. Pharmaceutical acceptable salts are well known in the art. Examples of pharmaceutical acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutical acceptable salts include adipate, alginate, ascorbate, aspartate, benzenesulfonate, benzoate, bisulphate, borate, butyrate, camphorate, camphorsulfonate, citrate, cyclopentanepropionate, digluconate, dodecylsulphate, ethanesulfonate, formate, fumarate, glucoheptonate, glycerophosphate, gluconate, hemisulphate, heptanoate, hexanoate, hydroiodide, 2-hydroxyethanesulfonate, lactobionate, lactate, laurate, lauryl sulphate, malate, maleate, malonate, methanesulfonate, 2-naphthalenesulfonate, nicotinate, nitrate, oleate, oxalate, palmitate, pamoate, pectinate, persulphate, 3-phenylpropionate, phosphate, picrate, pivalate, propionate, stearate, succinate, sulphate, tartrate, thiocyanate, p-toluenesulfonate, undecanoate, valerate salts.

Salts derived from appropriate bases include alkali metal, alkaline earth metal, and ammonium. Representative alkali or alkaline earth metal salts include sodium, lithium, potassium, calcium, magnesium. Further pharmaceutical acceptable salts include, when appropriate, nontoxic ammonium, quaternary ammonium, and amine cations formed using counterions such as halide, hydroxide, carboxylate, sulphate, phosphate, nitrate, lower alkyl sulfonate and aryl sulfonate.

Polymers of formula (I), (Ibis), (Iter) or (V) contain basic, e.g. amino, groups are capable of forming pharmaceutically acceptable salts with acids. Pharmaceutically acceptable acid addition salts of the polymers of formula (I), (Ibis), (Iter) or (V) may include, but are not limited to, those of inorganic acids such as hydrohalic acids (e.g. hydrochloric, hydrobromic and hydroiodic acid), sulfuric acid, nitric acid and phosphoric acids. Pharmaceutically acceptable acid addition salts of the polymers of formula (I), (Ibis), (Iter) or (V) may include, but are not limited to, those of organic acids such as aliphatic, aromatic, carboxylic and sulfonic classes of organic acids, examples of which include: aliphatic monocarboxylic acids such as formic acid, acetic acid, propionic acid or butyric acid; aliphatic hydroxy acids such as lactic acid, citric acid, tartaric acid or malic acid; dicarboxylic acids such as maleic acid or succinic acid; aromatic carboxylic acids such as benzoic acid, p-chlorobenzoic acid, phenylacetic acid, diphenylacetic acid or triphenylacetic acid; aromatic hydroxyl acids such as o-hydroxybenzoic acid, p-hydroxybenzoic acid, 1-hydroxynaphthalene-2-carboxylic acid or 3-hydroxynaphthalene-2-carboxylic acid; and sulfonic acids such as methanesulfonic acid, ethanesulfonic acid or benzenesulfonic acid. Other pharmaceutically acceptable acid addition salts of the polymers of formula (I), (Ibis), (Iter) or (V) include, but are not limited to, those of glycolic acid, glucuronic acid, furoic acid, glutamic acid, anthranilic acid, salicylic acid, mandelic acid, embonic (pamoic) acid, pantothenic acid, stearic acid, sulfanilic acid, algenic acid and galacturonic acid. Wherein the polymer of Formula I, Ibis or Iter comprises a plurality of basic groups, multiple centres may be protonated to provide multiple salts, e.g. di- or tri\-salts of compounds of formula (I), (Ibis), (Iter) or (V). For example, a hydrohalic acid salt of a polymer of formula (I), (Ibis), (Iter) or (V) as described herein may be a monohydrohalide, dihydrohalide or trihydrohalide, etc. The salts include, but are not limited to those resulting from addition of any of the acids disclosed above. In one embodiment of the polymer of formula (I), (Ibis), (Iter) or (V), two basic groups form acid addition salts. In a further embodiment, the two addition salt counterions are the same species, e.g. dihydrochloride, dihydrosulphide etc. Typically, the pharmaceutically acceptable salt is a hydrochloride salt, such as a dihydrochloride salt.

Polymers of formula (I), (Ibis), (Iter) or (V) which contain acidic, e.g. carboxyl, groups are capable of forming pharmaceutically acceptable salts with bases. Pharmaceutically acceptable basic salts of the polymers of formula (I), (Ibis), (Iter) or (V) may include, but are not limited to, metal salts such as alkali metal or alkaline earth metal salts (e.g. sodium, potassium, magnesium or calcium salts) and zinc or aluminium salts. Pharmaceutically acceptable basic salts of the polymers of formula (I), (Ibis), (Iter) or (V) may include, but are not limited to, salts formed with ammonia or pharmaceutically acceptable organic amines or heterocyclic bases such as ethanolamines (e.g. diethanolamine), benzylamines, N-methyl-glucamine, amino acids (e.g. lysine) or pyridine.

Hemisalts of acids and bases may also be formed, e.g. hemisulphate salts.

Pharmaceutically acceptable salts of polymers of Formula I, Ibis or Iter may be prepared by methods well-known in the art.

The polymers of formula (I), (Ibis), (Iter) or (V) may exist in both unsolvated and solvated forms. The term "solvate" includes molecular complexes comprising the polymer and one or more pharmaceutically acceptable solvent molecules such as water or C₁₋₆ alcohols, e.g. ethanol. The term "hydrate" means a "solvate" where the solvent is water.

The polymers may exist in solid states from amorphous through to crystalline forms. All such solid forms are included within the invention.

The polymers may exist in one or more geometrical, optical, enantiomeric, diastereomeric and tautomeric forms, including but not limited to *cis-* and trans-forms, E- and Z-forms, R-, *S*- and meso-forms, keto- and enol-forms. All such isomeric forms are included within the invention. The isomeric forms may be in isomerically pure or enriched form, as well as in mixtures of isomers (e.g. racemic or diastereomeric mixtures).

The invention includes pharmaceutically acceptable isotopically-labelled polymers of formula (I), (Ibis), (Iter) or (V) wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled polymers of formula (I), (Ibis), (Iter) or (V), for example, those incorporating a radioactive isotope, are useful in drug and/or substrate tissue distribution studies. The radioactive isotopes ³H and ¹⁴C are particularly useful for this purpose in view of their ease of incorporation and ready means of detection.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled polymers of formula (I), (Ibis), (Iter) or (V) can generally be prepared by conventional techniques known to those skilled in the art or by processes analogous to those described herein using an appropriate isotopically-labelled reagent in place of the non-labelled reagent previously employed.

It will be appreciated that the polymers, as described herein, may be substituted with any number of substituents or functional moieties. The terms substituted, whether preceded by the term "optionally" or not, and substituent, as used herein, refer to the ability, as appreciated by one skilled in this art, to change one functional group for another functional group provided that the valency of all atoms is maintained. When more than one position in any given structure may be substituted with more than one substituent selected from a specified group, the substituent may be either the same or different at every position. The substituents may also be further substituted (e.g., an aryl group substituent may have another substituent off it, such as another aryl group, which is further substituted with fluorine at one or more positions).

The term thiohydroxyl or thiol, as used herein, refers to a group of the formula -SH.

### Moiety having a zwitterionic polymer

In another embodiment of the first aspect of the invention, optionally in combination with any of the embodiments provided above or below, the moiety having a zwitterionic polymer is one wherein r represents 1.

In another embodiment of the first aspect of the invention, optionally in combination with any of the embodiments provided above or below, the moiety having a zwitterionic polymer is one where R'₄ represents an aryl, alkyl or -S-R₅, wherein R₅ is as defined above; particularly, R'₄ represents an aryl, alkyl, -S-aryl or -S-alkyl.

In another embodiment of the first aspect of the invention, optionally in combination with any of the embodiments provided above or below, the moiety having a zwitterionic polymer is one where R₉ represents -alkylene-C(O)-O-alkylene-, wherein the alkylene is optionally substituted (as defined in any of the above embodiments); particularly R₉ represents -(CR_{y}R'_{y})-(CH₂)ₛ-C(O)-O-(CH₂)ᵤ-*, wherein Ry and R'y are independently selected from hydrogen, halogen, trihalomethyl, trihaloethyl, -NO₂, -CN, -N⁺(C₁₋₆alkyl)₂O-, - CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, - C(=O)NH₂, -C₁₋₆alkyl, -C₃₋₆cycloalkyl, -C₃₋₆heterocycloalkyl, -Z^{u}C₁₋₆alkyl or-z^{u}-C₃₋₆cycloalkyl, wherein Z^{u} is defined above; * indicates the binding of R₉ to the -N- of the substituent of formula (i); and s and u are integers from 1 to 20. In another embodiment of the first aspect of the invention, optionally in combination with any of the embodiments provided above or below, Ry and R'y are independently selected from hydrogen, CN, and alkyl. In another embodiment of the first aspect of the invention, optionally in combination with any of the embodiments provided above or below, u represents from 1 to 10. In another embodiment of the first aspect of the invention, optionally in combination with any of the embodiments provided above or below, s represents from 1 to 10.

In another embodiment of the first aspect of the invention, optionally in combination with any of the embodiments provided above or below, the zwitterionic polymer PZ consists of zwitterionic monomers.

In another embodiment of the first aspect of the invention, optionally in combination with any of the embodiments provided above or below, the zwitterionic polymer PZ consists of from 2 to 100 monomers, particularly from 10 to 90 monomers, more particularly from 15 to 80 monomers.

The zwitterionic monomers can be derived from zwitterionic species with proton doning end groups (i.e. proton donors) or accepting end groups (i.e. proton acceptors) which are functionalized with polymerizable olefinic groups. In some cases, a zwitterionic group of a monomer can be formed by a carboxylic acid group, a sulfonic acid group, or a phosphoric acid group. In some cases, a monomer can include a zwitterionic group composed of an acrylate, a methacrylate, an acrylamide, or a methacrylamide. In some cases, a cation of a zwitterionic group can be formed by an (cyclo)aliphatic or aromatic amine, an amidine, or a guanidine. In some cases, a cation of a zwitterionic group can be a quaternary amine. In some cases, the proton donor end groups can be hydroxyl moieties. In some cases, proton accepting end groups can be amino moieties.

In another embodiment of the first aspect of the invention, optionally in combination with any of the embodiments provided above or below, the one or more zwitterionic monomers comprise(s) a positively charged nitrogen group and a negatively charged group distal to the positively charged nitrogen group on the zwitterion such that there is a separation by at least one carbon atom; particularly from 2 to 3 carbon atoms.

**In** some cases, a cation and an anion of a zwitterionic group can be part of the same pendant group of the monomer unit.

**In** one embodiment of the first aspect of the invention, optionally in combination with any of the embodiments provided above or below, the one or more zwitterionic monomers are betaine monomers, such as carboxybetaines, phosphobetaines and sulfobetaines. Illustrative non-limitative examples are carboxybetaine methacrylamide (CBMAA); sulfobetaine metacrylate; N,N-dimethyl-N-acryloyloxyethyl-N-(3-sulfopropyl)-ammonium betaine; N,N-dimethyl-N-acrylamidopropyl-N-(2-carboxymethyl)-ammonium betaine; N,N-dimethyl-N-acrylamidopropyl-N-(3-sulfopropyl)-ammonium betaine; N,N-dimethyl-N-acrylamidopropyl-N-(2-carboxymethyl)-ammonium betaine; 2-(methylthio)ethyl methacryloyl-S-(sulfopropyl)-sulfonium betaine; 1-(3-sulfopropyl)-2-vinylpyridinium betaine; N-(4-sulfobutyl)-N-methyl-N,N-diallylamine ammonium betaine (MDABS); and N,N-diallyl-N-methyl-N-(2-sulfoethyl)ammonium betaine. In one embodiment of the first aspect of the invention, optionally in combination with any of the embodiments provided above or below, the one or more monomers are the same or different and represent sulfobetaines.

Other suitable zwitterionic monomers that may be used to form zwitterionic include, but are not limited to, 2-Methacryloyloxyethyl phosphorylcholine; [3-(Methacryloylamino)propyl]dimethyl(3-sulfopropyl)ammonium hydroxide inner salt (also known as 3-(dimethyl{3-[(2-methylacryloyl)amino]propyl}ammonio)propane-1-sulfonate), [2-(Methacryloyloxy)ethyl]dimethyl-(3-sulfopropyl)ammonium hydroxide (also known as dimethyl-[2-(2-methylprop-2-enoyloxy)ethyl]-(3-sulfopropyl)azanium); 2-[(2-acryloylethyl)dimethylammonio]ethyl 2-methyl phosphate; 2-(acryloyloxyethyl)-2'-(trimethylammonium)ethyl phosphate; [(2-acryloylethyl)dimethylammonio]methyl phosphonic acid; 2-methacryloyloxyethyl phosphorylcholine (MPC); 2-[(3-acrylamidopropyl)dimethylammonio]ethyl 2'-isopropyl phosphate (AAPI); 1-vinyl-3-(3-sulfopropyl)imidazolium hydroxide; and (2-acryloxyethyl)carboxymethyl methylsulfonium chloride as well as any derivative, salt, copolymer and combinations thereof.

**In** one embodiment of the first aspect of the invention, optionally in combination with any of the embodiments provided above or below, the zwitterionic polymer PZ is a homopolymer, which means that it is composed by the same zwitterionic monomer. In an alternative embodiment of the first aspect of the invention, optionally in combination with any of the embodiments provided above or below, the zwitterionic polymer PZ is a heteropolymer, which means that it is composed of two or more different zwitterionic monomers, creating random-, block-, or alternating-copolymer systems.

In some cases, zwitterionic monomers can be used to prepare linear architectures, combs, stars, brushes, hyperbranched/arborescent, and crosslinked gel systems may also be used. The length of the graft and density of the graft chains can be optimized.

### Oligopeptide

In one embodiment of the first aspect of the invention, optionally in combination with any of the embodiments provided above or below, R₁ and R₂ are both oligopeptides.

In another embodiment of the first aspect of the invention, optionally in combination with any of the embodiments provided above or below, R₁ and R₂ represent the same oligopeptide.

According to the present invention, an "oligopeptide" comprises a string of at least three amino acids linked together by peptide bonds. Such peptides can contain only natural amino acids, although non-natural amino acids (i.e., compounds that do not occur in nature but that can be incorporated into a polypeptide chain) and/or amino acid analogues as are known in the art may alternatively be employed. Also, one or more of the amino acids in such peptides may be modified, for example, by the addition of a chemical entity such as a carbohydrate group, a phosphate group, a farnesyl group, an isofarnesyl group, a fatty acid group, or a linker for conjugation, functionalization, or other modification, etc.

In one embodiment of the first aspect of the invention, optionally in combination with any of the embodiments provided above or below, the oligopeptides in the polymers defined herein typically comprise from 3 to 20 amino acid residues, particularly from 3 to 10 amino acid residues, more particularly from 3 to 6 amino acid residues. Alternatively, the oligopeptides in the polymers defined herein may comprise from 4 to 20 amino acid residues, particularly from 4 to 10 amino acid residues, more particularly from 4 to 6 amino acid residues.

In one embodiment of the first aspect of the invention, optionally in combination with any of the embodiments provided above or below, the or each oligopeptide may be hydrophobic. The or each oligopeptide may comprise naturally occurring amino acids that are hydrophobic such as valine, leucine, isoleucine, methionine, tryptophan, phenylalanine, cysteine, tyrosine and alanine; in particular, the or each oligopeptide may comprise valine, leucine, isoleucine, methionine, tryptophan and phenylalanine.

In another embodiment of the first aspect of the invention, optionally in combination with any of the embodiments provided above or below, the or each oligopeptide may be hydrophilic. The or each oligopeptide may comprise naturally occurring amino acids that are hydrophilic such as serine, threonine, cysteine, asparagine and glutamine, and may further comprise naturally occurring amino acids that are charged at pH7.

In another embodiment of the first aspect of the invention, optionally in combination with any of the embodiments provided above or below, the or each oligopeptide has a net positive charge at pH 7. The or each oligopeptide may comprise naturally occurring amino acids that are positively charged at pH 7, that is, lysine, arginine and histidine. In one embodiment of the first aspect of the invention, optionally in combination with any of the embodiments provided above or below, the or each oligopeptide consists of homooligopeptides consisting of naturally occurring amino acids that are positively charged at pH 7, that is, lysine, arginine and/or histidine. For example, the or each oligopeptide may be selected from the group consisting of polylysine, polyarginine, and polyhistidine, each of which may be terminated with cysteine. I

In another embodiment of the first aspect of the invention, optionally in combination with any of the embodiments provided above or below, the or each oligopeptide is a compound of Formula III: 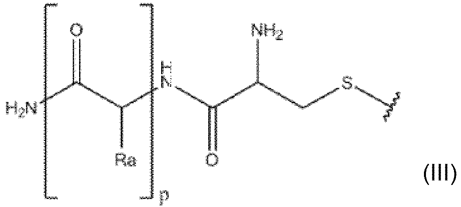 wherein p is an integer from 2 to 19, typically from 3 to 9 or from 3 to 5, and wherein Rₐ is selected at each occurrence from the group consisting of H₂NC(=NH)-NH(CH₂)₃-, H₂N(CH₂)₄-, and (1*H*-imidazol-4-yl)-CH₂-; and the wavy line points out that the oligopeptide is bound to the pBAE backbone through -S-.

Where the or each oligopeptide is a compound of Formula III, the L₁ and/or L₂ (and/or L₁, when present) linking the or each oligopeptide to the polymer is a bond and the terminal cysteine residue provides a means of coupling the or each oligopeptide to the acrylate terminated compound of the pBAE backbone. The thiol functionality provides faster, more efficient and more easily controlled addition to the double bond. By contrast, where the or each oligopeptide is terminated in an amine functionality for coupling, an excess of this compound is required in the coupling step.

Alternatively, the or each oligopeptide may have a net negative charge at pH 7. The or each oligopeptide may comprise naturally occurring amino acids that are negatively charged at pH 7, that is, aspartic acid and glutamic acid. For example, the or each oligopeptide may be selected from polyaspartic acid and polyglutamic acid, each of which may be terminated with cysteine. In this embodiment, the or each oligopeptide may be a compound of Formula IIIbis 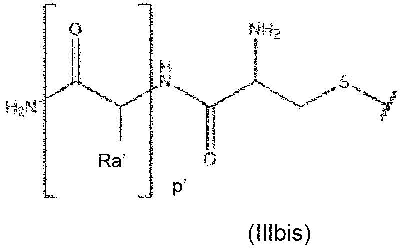 wherein p' is an integer from 2 to 19, typically from 3 to 9 or from 3 to 5, and wherein Rₐ' is HO₂C(CH₂)₂- or HO₂C-CH₂-. In this case, the L₁ and/or L₂ linking the or each oligopeptide to the polymer is a bond as the terminal cysteine residue provides a means of coupling the or each oligopeptide to the acrylate terminated pBAE backbone.

Alternatively, the or each oligopeptide may comprise a mixture of naturally occurring amino acids that are negatively charged at pH 7 and naturally occurring amino acids that are positively charged at pH 7.

### Cell-targeting moiety

In another embodiment of the first aspect of the invention, optionally in combination with any of the embodiments provided above or below, the polymer OM-pZ-pBAE of the invention further includes one or more cell-targeting moieties.

In the context of the present invention, the term "cell-targeting moiety" means a moiety that binds a cell. In certain embodiments, cell-targeting ligands are selective for one or more particular cell type. In certain embodiments, cell-targeting moieties selectively bind a receptor, such as a cell surface receptor.

Illustrative non-limitative examples of cell-targeting moieties include, but are not limited to, an antibody, a receptor ligand, a hormone, a vitamin, and an antigen, however, the present invention is not limited by the nature of the targeting agent. In some embodiments, the cell-targeting moiety is a receptor ligand, such as a ligand for CFTR, EGFR, the estrogen receptor, FGR2, folate receptor, IL-2 receptor, glycoprotein, the retinol binding protein and VEGFR.

In another embodiment of the first aspect of the invention, optionally in combination with any of the embodiments provided above or below, the receptor ligand is a carbohydrate (such as a lectine) or a sugar (such as mannose or galactose).

In another embodiment, optionally in combination with any of the embodiments provided above or below, the cell-targeting moiety comprises a targeting group linker.

In another embodiment, optionally in combination with any of the embodiments provided above or below, the cell-targeting group linker comprises one or more groups selected from among: phosphate, amide, ether, ester, pyrrolidine, disulfide, and methylene.

In another embodiment, optionally in combination with any of the embodiments provided above or below, the cell-targeting moiety is bound to R₃. The binding of the cell-targeting moiety to R₃ can be performed following well-known protocols. The skilled person, using their general knowledge, is able to select the most appropriate conditions/reagents, depending on the particular cell-targeting moiety and the particular meaning of R₃. For example, the cell-targeting moiety can be firstly modified to incorporate a thiol group, preferably at a terminal position, and then, reacting the thiol derivative with the polymer of formula (I), under appropriate conditions to promote the generation of a -S-S-. The reaction can be based on the aminolysis on the Z group of the compound of formula (IV) by preparing a thiol-derivative which reacts with the cell-targeting moiety previously thiol-functionalized. Reaction conditions and reagents useful to perform the aminolysis are widely known for those skilled in the art (Hess A. et al., 2020; Boyer C. et al., 2009).

An example of the appropriate conditions to allow the derivatization are provided below, wherein the polymer of formula (I) is modified to include retinol as cell-targeting moiety.

### Nanoparticles

The present invention further provides nanoparticles comprising one or more polymers of formula (I) as defined above. All the embodiments provided above for the polymers of the first aspect of the invention, are also embodiments of the nanoparticles of the fourth aspect of the invention.

Such nanoparticles are suitable for encapsulation of a particular molecule (compound of interest), such as a diagnostic and imaging reagents or an active agent, and show improved pharmacological properties.

Diagnostic and imaging agents include contrast agents, magnetic materials, agents sensitive to light, radiolabels, and fluorescent compounds, such as carboxyfluorescein. Such agents may be used for biodistribution studies *in vitro* and *in vivo.* Delivery of nanoparticles of the present invention to the brain has been demonstrated by such studies. For example, paclitaxel-loaded nanoparticles comprising surface-modifying agents have been detected in the brain in biodistribution studies *in vivo.* Moreover, fluorescently labelled nanoparticles can be used in a cellular study simulating the blood-brain barrier.

The active agent(s) may be present within the nanoparticles or on the surfaces of the nanoparticles. Typically the active agent is present within the nanoparticles. The interaction between the active agent(s) and the nanoparticle is typically non-covalent, for example, hydrogen bonding, electrostatic interaction or physical encapsulation. Typically the interaction is electrostatic

The nanoparticles are biocompatible and sufficiently resistant to their environment of use that a sufficient amount of the nanoparticles remain substantially intact after entry into the mammalian body so as to be able to reach the desired target and achieve the desired physiological effect. The polymers described herein are biocompatible and preferably biodegradable.

Herein, the term 'biocompatible' describes as substance which may be inserted or injected into a living subject without causing an adverse response. For example, it does not cause inflammation or acute rejection by the immune system that cannot be adequately controlled. It will be recognized that "biocompatible" is a relative term, and some degree of immune response is to be expected even for substances that are highly compatible with living tissue. An in vitro test to assess the biocompatibility of a substance is to expose it to cells; biocompatible substances will typically not result in significant cell death (for example, >20%) at moderate concentrations (for example, 29 µg/10<4 >cells).

Herein, the term 'biodegradable' describes a polymer which degrades in a physiological environment to form monomers and/or other non-polymeric moieties that can be reused by cells or disposed of without significant toxic effect. Degradation may be biological, for example, by enzymatic activity or cellular machinery, or may be chemical, typically a chemical process that takes place under physiological conditions. Degradation of a polymer may occur at varying rates, with a half-life in the order of days, weeks, months, or years, depending on the polymer or copolymer used. The components preferably do not induce inflammation or other adverse effects in vivo. In certain preferred embodiments, the chemical reactions relied upon to break down the biodegradable compounds are uncatalysed.

Herein, the term "nanoparticles" refers to a solid particle with a diameter of from about 1 to about 1000 nm, particularly from 50 to 800nm, particularly from 100 to 500 nm. The mean diameter of the nanoparticles of the present invention may be determined by methods known in the art, preferably by dynamic light scattering. In particular, the invention relates to nanoparticles that are solid particles with a diameter of from about 1 to about 1000nm when analysed by dynamic light scattering at a scattering angle of 173° and at a temperature of 25°C, using a sample appropriately diluted with filtered water and a suitable instrument such as the Zetasizer^{™} instruments from Malvern Instruments (UK).

Where a particle is said to have a diameter of x nm, there will generally be a distribution of particles about this mean, but at least 50% by number (e.g. >60%, >70%, >80%, >90%, or more) of the particles will have a diameter within the range x±20%.

Nanoparticles of the present disclosure may be formed with high active agent content and high encapsulation efficiency, as explained above and showed below.

Herein, the active agent encapsulation efficiency refers to the active agent incorporated into the nanoparticles as a weight percentage of the total active agent used in the method of preparation of the active agent-containing nanoparticles. It is typically up to and including 95%, more typically from 70% to 95%.

Herein, active agent entrapment refers to the weight percentage of the active agent in the active agent-loaded nanoparticles. Active agent entrapment is preferably at least 2 wt %, more preferably at least 5 wt %, more preferably at least 10 wt % and typically in the range of from 2 wt % to 20 wt %, more preferably from 5 wt % to 20 wt %, more preferably from 10 wt % to 20 wt %.

In one embodiment of the fourth aspect of the invention, optionally in combination with any of the embodiments provided above or below, the compound of interest is an active ingredient.

In one embodiment of the fourth aspect of the invention, optionally in combination with any of the embodiments provided above or below, the compound of interest, particularly the active ingredient, is negatively charged at biological pH.

In an alternative embodiment of the fourth aspect of the invention, optionally in combination with any of the embodiments provided above or below, the compound of interest, particularly the active ingredient, is positively charged at biological pH.

The "positively charged at biological pH" in the context of the invention is meant to include any compound, particularly any pharmacologically active substance, that bear negative charges in the molecule in an aqueous solution at a biological pH (about 7).

The "negatively charged at biological pH" in the context of the invention is meant to include any compound, particularly any pharmacologically active substance, that bear negative charges in the molecule in an aqueous solution at a biological pH (about 7). In one embodiment, the anionic nature can be imparted from one or more functional groups selected from the group consisting of carboxyl, phosphate and sulphate groups.

In one embodiment of the present invention, optionally in combination with any of the embodiments provided above or below, the negatively charged compound of interest is a polyanionic small molecule, a nucleic acid, polypeptide or protein. As used herein, the term "small molecule" refers to organic compounds, whether naturally-occurring or artificially created (e.g., via chemical synthesis) that have relatively low molecular weight and that are not proteins, polypeptides, or polynucleotides. Typically, small molecules have a molecular weight of less than about 1500 g/mol. Examples of the polyanionic small molecules may include heparin, calcitonin, etc.

The nucleic acid may be a nucleic acid drug, such as deoxyribonucleic acid, ribonucleic acid, or a polynucleotide derivative wherein the backbone, sugar or base is chemically modified or the end of the nucleic acid is modified. Particularly, it may be one or more nucleic acid selected from the group consisting of RNA, DNA (such as a gene or oligonucleotide), siRNA (small interfering RNA), an aptamer, antisense oligodeoxynucleotide (ODN), antisense RNA, ribozyme, DNAzyme, ASO, ribozyme, oligonucleotide RNA inhibitor, immune modulating oligonucleotide or other desired negatively charged nucleic acid.

Also, in order to increase the stability of the nucleic acid in blood or weaken the immune response, the backbone, sugar or base of the nucleic acid may be chemically modified or the end of the nucleic acid may be modified. Specifically, a portion of the phosphodiester bond of the nucleic acid may be substituted by a phosphorothioate or boranophosphate bond, or the nucleic acid may include at least one nucleotide wherein various functional groups such as a methyl group, a methoxyethyl = 30 group or fluorine are introduced in the 2'-OH position of some riboses. Therefore, the polynucleotide may be derived from natural nucleosides (i.e., adenosine, thymidine, guanosine, cytidine, uridine, inosine, xanthosine, deoxyadenosine, deoxythymidine, deoxyguanosine, deoxyinosine, and deoxycytidine), nucleoside analogues (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), or mixtures thereof. The nucleotides may be derived from chemically modified bases, biologically modified bases (e.g., methylated bases), intercalated bases, unmodified or modified sugars (e.g., 2'-fluororibose, ribose, 2'-deoxyribose, arabinose, and hexose), and/or unmodified or modified phosphate groups (e.g., phosphorothioates and 5'-N-phosphoramidite linkages).

Typically, a polynucleotide comprises at least three nucleotides. Preferably, the polynucleotide is 20-30 nucleotides in length, more preferably 20-25 nucleotides in length, for example, 22 nucleotides in length.

In another embodiment, optionally in combination with any of the embodiments provided above or below, one or more ends of the nucleic acid may be modified with one or more selected from the group consisting of cholesterol, tocopherol, a fatty acid having 10-24 carbon atoms, and any analogue, derivative and metabolite thereof. For example, siRNA may be modified at the 5' end or at the 3' end or at both ends of the sense and/or antisense strand, and preferably at the end of the sense strand.

In another embodiment, optionally in combination with any of the embodiments provided above or below, the nanoparticle comprises a polymeric shell comprising the polymer(s) of formula (I); and a core comprising the compound(s) encapsulated therein.

In another embodiment, optionally in combination with any of the embodiments provided above or below, the nanoparticle may comprise one or more polymers of formula (Iter): 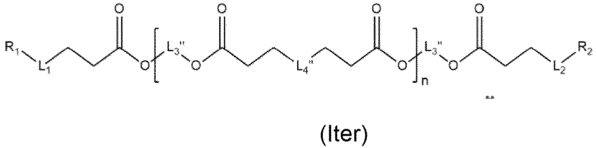
L"₃ is independently selected from the group consisting of alkylene, alkenylene, heteroalkylene, heteroalkenylene, arylene, and heteroarylene; or, alternatively at least one of L₃" is
wherein
   T₁" is 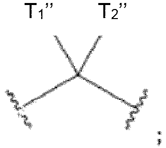 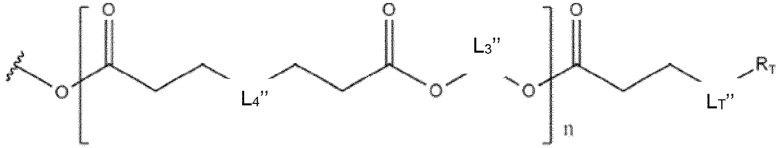
   and T₂" is selected from H, alkyl, and 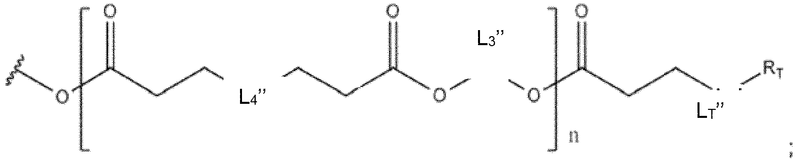
   wherein L_{T}" is independently selected from the group consisting of: 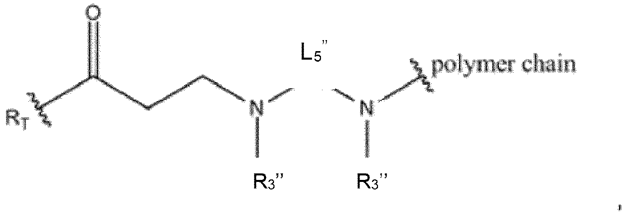
   O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl, and
the remaining L₃" groups are independently selected at each occurrence from the group consisting of alkylene, alkenylene, heteroalkylene, heteroalkenylene, arylene, and heteroarylene;
L"₄ is independently selected from the group consisting of (v) and (vi) 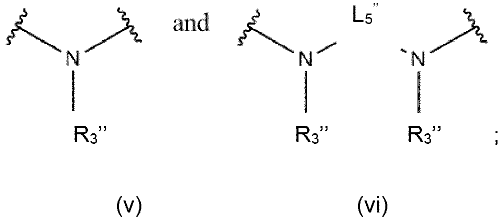
L₅" is independently selected from the group consisting of alkylene, alkenylene, heteroalkylene, heteroalkenylene, arylene, and heteroarylene;
each R₃" is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), and a moiety having an amine group (-NH₂),
wherein:
   - said polyalkylene glycol is either bound directly to the nitrogen atom to which R₃" is attached or bound to the nitrogen atom to which R₃" is attached via a linker moiety, wherein said linker moiety is an alkylene, cycloalkylene, alkenylene, cycloalkenylene, heteroalkylene, heterocycloalkylene, arylene or heteroarylene group;
   - the moiety having a hydroxyl group (-OH) is selected from the group consisting of - (CH₂)ₚOH, and -(CH₂)₂(OCH₂CH₂)_{q}OH; and
   - the moiety having an amino group (-NH₂) is selected from the group consisting of -(CH₂)ₚNH₂, and -(CH₂)₂(OCH₂CH₂)_{q}NH₂;
n is an integer from 5 to 1000, p is an integer from 1 to 20, and q is an integer from 1 to 10,
each L₁ and L₂ is independently selected from the group consisting of: 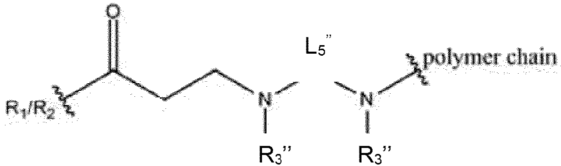
O, S, NRₓ, and a bond; wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl; and R₃" and L₅" being as defined above; and
R₁, R₂ and R_{T} are independently selected from an oligopeptide and R_{y},
R_{y} being selected from the group consisting of hydrogen, halogen, alkyl, cycloalkyl, alkenyl, cycloalkenyl, heteroalkyl, heterocycloalkyl, acyl, aryl, and heteroaryl; and provided that at least one of R₁, R₂ and R_{T} is an oligopeptide.

In one embodiment, optionally in combination with any of the embodiments provided above, or below, the polymer of formula (Iter) is one wherein L₃" presents alkylene.

In one embodiment, optionally in combination with any of the embodiments provided above, or below, the polymer of formula (Iter) is one wherein L₄" presents a group of formula (v), wherein R₃" is as defined above.

In one embodiment, optionally in combination with any of the embodiments provided above or below, the polymer of formula (Iter) is one of formula (v) wherein R"₃ groups are the same or different and are selected from -(CH₂)ₚOH, -(CH₂)₂(OCH₂CH₂)_{q}OH, -(CH₂)ₚNH₂, and -(CH₂)₂(OCH_{2C}H₂)_{q}NH₂, wherein p and q are as defined above.

All the embodiments provided under the first aspect of the invention for the polymer of formula (I), regarding L₁, L₂, R₃ and R₂, including the oligopeptides useful in the context of the invention, are also embodiments of the polymer of formula (Iter).

In another embodiment, optionally in combination with any of the embodiments provided above or below, the polymer of formula (Iter) is one wherein:
R₁ and R₂ are oligopeptides as defined above, particularly having negative charge at pH 7;
L₁ and L₂ represent a bond;
L₃" represents alkylene; and
L₄" represents a substituent of formula (i), wherein R₃" are the same or different and are selected from -(CH₂)ₚOH, -(CH₂)₂(OCH₂CH₂)_{q}OH, -(CH₂)ₚNH₂, and -(CH₂)₂(OCH₂CH₂)_{q}NH₂.

As it can be derived from Table 8 below, the incorporation of one or more polymers of formula (Iter), further reduced the average size of the resulting polyplexes.

In another embodiment, optionally in combination with any of the embodiments provided above or below, the nanoparticle comprises a polymeric shell comprising the polymer(s) of formula (I) and one or more polymer(s) of formula (Iter); and a core comprising the compound(s) encapsulated therein.

In one embodiment, optionally in combination with any of the embodiments provided above or below, the nanoparticle may comprise a negatively charged compound of interest, as defined above, and one or more polymers of formula (I) and optionally one or more polymers of formula (Iter), having a net positive charge at pH 7. The positively charged oligopeptides interact with negatively charged compound during the process of nanoparticle formation and facilitate encapsulation of the compound in the nanoparticles.

In an alternative embodiment, optionally in combination with any of the embodiments provided above or below, the nanoparticles may comprise polymers of formula (I) and optionally polymers of formula (Iter) wherein the or each oligopeptide has a net negative charge at pH 7 and a compound of interest having a net positive charge at pH7. The negatively charged oligopeptides interact with positively charged compound during the process of nanoparticle formation and facilitate encapsulation of the compound in the nanoparticles.

The present invention further provides a method of encapsulating a compound of interest, such as an active ingredient, in a matrix of polymers of formula I and optionally of formula (Iter) to form nanoparticles, the method comprising steps of: providing a charged compound of interest; providing the polymer(s) as defined in any of the above embodiments; and contacting the agent and the polymer under suitable conditions to form nanoparticles. The skilled person, using their general knowledge, can routinely adjust the parameters to obtain the nanoparticles. An illustrative example is provided below, in the Examples.

### Pharmaceutical Compositions

The compositions of the invention comprise a therapeutically effective amount of the nanoparticles of the invention, when they incorporate an active ingredient, together with pharmaceutically acceptable excipients and/or carriers.

The expression "therapeutically effective amount" as used herein, refers to the amount of the nanoparticles of the invention that, when administered passes to blood stream and is sufficient to prevent development of, or alleviate to some extent, one or more of the symptoms of the disease which is addressed. The particular dose of the nanoparticles administered according to this invention will of course be determined by the particular circumstances surrounding the case, including the compound administered, the route of administration, the particular condition being treated, and the similar considerations.

The term "pharmaceutically acceptable" refers to that excipients or carriers suitable for use in the pharmaceutical technology for preparing compositions with medical use.

The expression "excipients and/or carriers" refers to acceptable materials, compositions or vehicles. Each component must be pharmaceutically acceptable in the sense of being compatible with the other ingredients of the composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants.

The formulations of the pharmaceutical compositions described herein may be prepared by any method known or hereafter developed in the art of pharmacology. In general, such preparatory methods include the step of bringing the nanoparticles into association with an excipient and/or one or more other accessory ingredients, and then, if necessary and/or desirable, shaping and/or packaging the product into a desired single- or multidose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the nanoparticles.

The relative amounts of the active ingredient (i.e., the peptide as defined in any of the previous aspects and embodiments), the acceptable excipients, and/or any additional ingredients in the composition of the invention will vary, depending upon the identity, size, and/or condition of the subject treated and further depending upon the route by which the composition is to be administered.

Acceptable excipients used in the manufacture of these compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

### Obtaining of the polymers of formula (1)

The obtaining of this new family of polymers can involve the synthesis of acrylate-terminated polymers (pBAE), the modification of the acrylate ends with oligopeptides and the subsequent modification of the hydroxyl group of the side chain with the Chain Transfer Agent (CTA). Through a final grafting process of the zwitterionic monomer on the pBAE backbone.

As pointed out above, the present invention provides, in a second aspect, a process for obtaining the polymer of formula (I) which comprises the **RAFT** polymerization of a polymer of formula (Ibis), as defined above, which is characterized by incorporating at least one moiety comprising a CTA compound corresponding to a thio-based compound of formula (IV), together with one or more zwitterionic monomers, in the presence of a radical initiator. The skilled person, using their general knowledge will be able to routinary choose the more appropriate conditions to perform the polymerization. An illustrative example of the appropriate reaction conditions is provided below.

In one embodiment of the second aspect of the invention, optionally in combination with any of the embodiments provided above or below, the polymer (Ibis) is one wherein L₁' represents a bond.

In one embodiment of the second aspect of the invention, optionally in combination with any of the embodiments provided above or below, the polymer (Ibis) is one wherein L₂' represents a bond.

In one embodiment of the second aspect of the invention, optionally in combination with any of the embodiments provided above or below, the polymer (Ibis) is one wherein L₃' represents alkylene.

In one embodiment of the second aspect of the invention, optionally in combination with any of the embodiments provided above or below, the polymer (Ibis) is one wherein L₄' represents a group of formula (iii).

In one embodiment of the second aspect of the invention, optionally in combination with any of the embodiments provided above or below, the polymer (Ibis) is one wherein at least a 5% of the R'₃ groups in the polymer of formula (Ibis) represents a moiety of formula (IV) as defined above; particularly at least a 30%, 40%, 50%, 60%, 70%, 70%, 80% or 90% of the R'₃ groups in the polymer of formula (Ibis) represents a moiety of formula (IV) as defined above; particularly at least a 40% of the R'₃ groups in the polymer of formula (Ibis) represents a moiety of formula (IV) as defined above; particularly from 30 to 80%; particularly from 40 to 70% of the R'₃ groups in the polymer of formula (Ibis) represents a moiety of formula (IV) as defined above.

In one embodiment of the second aspect of the invention, optionally in combination with any of the embodiments provided above or below, the polymer (Ibis) is one wherein the remaining R'₃ groups, which are other than a moiety of formula (IV), are the same or different and are selected from -(CH₂)ₚOH, -(CH₂)₂(OCH₂CH₂)_{q}OH, -(CH₂)ₚNH₂, and -(CH₂)₂(OCH_{2C}H₂)_{q}NH₂, wherein p and q are as defined above.

In one embodiment of the second aspect of the invention, optionally in combination with any of the embodiments provided above or below, the polymer (Ibis) is one wherein the thio-based compound (IV) is one where Z represents an aryl, alkyl or -S-R'₅, wherein R'₅ is as defined above; particularly, R'₅ represents an aryl, alkyl, -S-aryl or -S-alkyl.

In one embodiment of the second aspect of the invention, optionally in combination with any of the embodiments provided above or below, the polymer (Ibis) is one wherein the thio-based compound (IV) is one where R'₉ represents -alkylene-C(O)-O-alkylene-, wherein the alkylene is optionally substituted; particularly R'₉ represents -(CR_{w}R'_{w})-(CH₂)ᵥ-C(O)-O-(CH₂)_{w}-*, wherein R_{w} and R'_{w} are independently selected from hydrogen, hydrogen, halogen, trihalomethyl, trihaloethyl, -NO₂, -CN, -N⁺(C₁₋₆alkyl)₂O-, -CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl and -z^{u}-C₃₋₆cycloalkyl, wherein Z^{u} is defined above; * indicates the binding of R'₉ to the -N- of the substituent of formula (i); and v and w are integers from 1 to 20.

In another embodiment of the second aspect of the invention, optionally in combination with any of the embodiments provided above or below, the polymer of formula (Ibis) is one wherein R_{w} and R'_{w} are independently selected from hydrogen, CN and alkyl.

In another embodiment of the second aspect of the invention, optionally in combination with any of the embodiments provided above or below, the polymer of formula (Ibis) is one wherein the thio-based compound (IV) is one where v is from 1 to 10.

In another embodiment of the second aspect of the invention, optionally in combination with any of the embodiments provided above or below, the polymer of formula (Ibis) is one wherein the thio-based compound (IV) is one wherein w is from 1 to 10.

In another embodiment of the second aspect of the invention, optionally in combination with any of the embodiments provided above or below, the polymer of formula (Ibis) is one wherein the oligopeptide(s) are as defined in any of the previous embodiments.

In one embodiment of the second aspect of the invention, optionally in combination with any of the embodiments provided above or below, the polymer (Ibis) is one wherein:
R₁ and R₂ are oligopeptides as defined in any of the embodiments provided above, under the first aspect of the invention, particularly negatively charged olipeptides at pH 7;
L₁'and L₂' represent a bond;
L₃' represents alkylene;
L₄' represents a substituent of formula (iii), wherein:
   - at least 5% of the R₃' groups represent a moiety of formula (IV) as defined above; Z being selected from alkyl, aryl, -S-alkyl, and -S-aryl; and R'₉ representing
   - alkylene-C(O)-O-alkylene-, wherein the alkylene is optionally substituted; and
   - the remaining R₃' groups, which are other than the moiety of formula (II), are the same or different and are selected from -(CH₂)ₚOH, -(CH₂)₂(OCH₂CH₂)_{q}OH, -(CH₂)ₚNH₂, and -(CH₂)₂(OCH₂CH₂)_{q}NH₂.

The RAFT polymerization is performed using zwitterionic monomer(s). Suitable zwitterionic monomers in the context of the invention are as they have been defined in above.

As the skilled person knows, the RAFT polymerization requires the presence of a radical initiator. Any of the well-known radical initiators suitable in the context of the RAFT polymerization can be used to polymerize the zwitterionic monomers in the CTA-based moiety of formula (IV). Illustrative non-limitative examples are azo polymerization initiators such as azobisisobutyronitrile (AIBN), 4,4'-azobis(4-cyanovaleric acid) (ACVA), also called 4,4'-azobis(4-cyanopentanoic acid), which are widely used, but also other azo polymerization initiators manufactured by companies such as Fujifilm or Otsuka Chemical Co. Ltd. (series of azo polymerization initiators "V").

In one embodiment of the process of the second aspect of the invention, optionally in combination with any of the embodiments provided above or below, the RAFT polymerization is performed at room temperature.

In one embodiment of the process of the second aspect of the invention, optionally in combination with any of the embodiments provided above or below, the RAFT polymerization is performed under agitation.

The polymer of formula (Ibis), which is characterized by being activated to perform the RAFT polymerization (due to the CTA moiety), can be obtained using well-known protocols. For example, they can be obtained by: (1) reacting a polymer of formula (V) or a pharmaceutical salt thereof with any suitable chain transfer agent, as defined above; and (2) the resulting polymer is then reacted with the oligopeptides.

The pBAE polymer of formula (V) is one wherein: 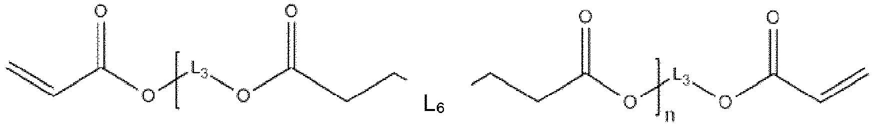
wherein L₃ is independently selected from the group consisting of alkylene, alkenylene, heteroalkylene, heteroalkenylene, arylene, and heteroarylene; or at least one occurrence of L₃ is 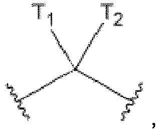
wherein T₁ is 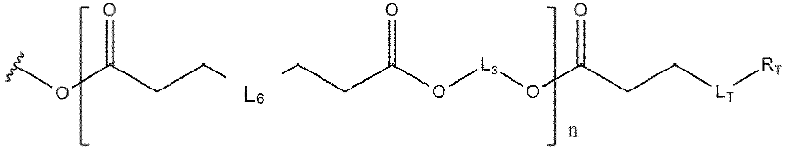
and T₂ is selected from H, alkyl, and 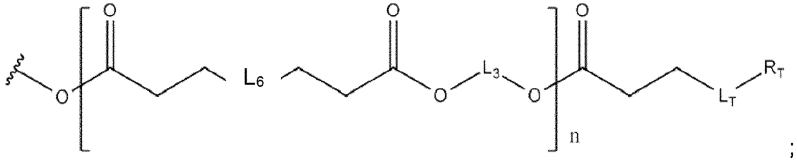
wherein L_{T} is independently selected from the group consisting of: 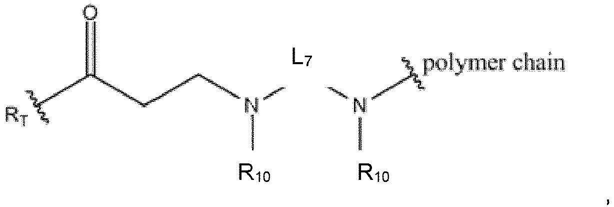
O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl, and the remaining L₃ groups are independently selected at each occurrence from the group consisting of alkylene, alkenylene, heteroalkylene, heteroalkenylene, arylene, and heteroarylene;
L₆ is independently selected from the group consisting of (vii) and (viii) 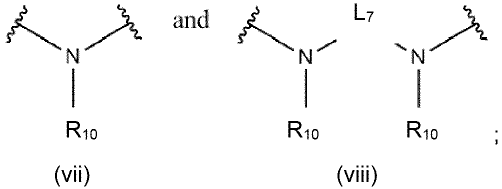
L₇ **is independently** selected from the group consisting of alkylene, alkenylene, heteroalkylene, heteroalkenylene, arylene, and heteroarylene;
R_{T} is independently selected from an oligopeptide and R_{y};
and wherein R_{y} is selected from the group consisting of hydrogen, halogen, alkyl, cycloalkyl, alkenyl, cycloalkenyl, heteroalkyl, heterocycloalkyl, acyl, aryl, and heteroaryl;
each R₁₀ is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), and a moiety having an amine group (-NH₂),; provided that at least one or more of the R₁₀ are moieties having a a hydroxyl group, wherein:
   - said polyalkylene glycol is either bound directly to the nitrogen atom to which R₃ is attached or bound to the nitrogen atom to which R₃ is attached via a linker moiety, wherein said linker moiety is an alkylene, cycloalkylene, alkenylene, cycloalkenylene, heteroalkylene, heterocycloalkylene, arylene or heteroarylene group;
   - the moiety having a hydroxyl group (-OH) is selected from the group consisting of -(CH₂)ₚOH, and -(CH₂)₂(OCH₂CH₂)_{q}OH;
   - the moiety having an amino group (-NH₂) is selected from the group consisting of -(CH₂)ₚNH₂, -(CH₂)₂(OCH₂CH₂)_{q}NH₂;
n is an integer from 5 to 1,000, p is an integer from 1 to 20, and q is an integer from 1 to 10.

In one embodiment, optionally in combination with any of the embodiments provided above or below, the polymer (V) is one wherein L₃ represents alkylene.

In one embodiment, optionally in combination with any of the embodiments provided above or below, the polymer (V) is one wherein L₆ represents a group of formula (vii).

In one embodiment, the polymer (V) is one wherein at least a 5% of the R₁₀ groups in the polymer of formula (V) represents a moiety having a hydroxyl group (-OH) as defined above; particularly at least a 30%, 40%, 50%, 60%, 70%, 70%, 80% or 90% of the R₁₀ groups in the polymer of formula (V) represents a moiety having a hydroxyl group (-OH) as defined above; particularly at least a 40% of the R₁₀ groups in the polymer of formula (V) represents a moiety having a hydroxyl group (-OH) as defined above; particularly from 30 to 80%; particularly from 40 to 70% of the R₁₀ groups in the polymer of formula (V) represents a moiety having a hydroxyl group (-OH) as defined above.

In one embodiment, the polymer (V) is one wherein at least a 5% of the R₁₀ groups in the polymer of formula (V) represents a moiety having a hydroxyl group (-OH) as defined above and the remaining R₁₀ groups present a moiety having an amino group as defined above; particularly at least a 30%, 40%, 50%, 60%, 70%, 70%, 80% or 90% of the R₁₀ groups in the polymer of formula (V) represents a moiety having a hydroxyl group (-OH) as defined above and the remaining R₁₀ groups present a moiety having an amino group as defined above; particularly at least a 40% of the R₁₀ groups in the polymer of formula (V) represents a moiety having a hydroxyl group (-OH) as defined above and the remaining R₁₀ groups present a moiety having an amino group as defined above; particularly from 30 to 80%; particularly from 40 to 70% of the R₁₀ groups in the polymer of formula (V) represents a moiety having a hydroxyl group (-OH) as defined above and the remaining R₁₀ groups present a moiety having an amino group.

The compound of formula (V) is firstly "activated" by performing a sterification reaction between the one or more -OH of the R₁₀ groups and the particular CTA, thus obtaining a "CTA-modified pBAE". Appropriate reaction conditions can be routinary optimized by the skilled person. The present invention provides illustrative reaction conditions in the Examples below, using two different CTAs.

And, subsequently, the resulting CTA-modified pBAE of formula reacts with the corresponding oligopeptide(s) which are as defined in any of the above embodiments, in the presence of a polar solvent, such as DMSO, at room temperature and agitation, thus obtaining the polymer of formula (Ibis). Illustrative reaction conditions to perform the end modification of the CTA-modified pBAE with the oligopeptides have been widely disclosed, such as in US20180250410. Further exemplary conditions are also provided in Examples below.

The obtaining of polymers of formula (V) has been widely disclosed in the prior art, for example in the US2018025410.

An alternative process to the one of the second aspect of the invention is based on reacting the pBAE of formula (Iter), wherein one or more of the L₄" represents (v) and one or more of the R₃" represents a moiety having a hydroxyl group (-OH), as defined above and the zwitterionic polymeric chain, previously formed by RAFT polymerization or any other appropriate polymerization technique.

The present invention also provides a poly(beta-aminoester) polymer of formula (I) or a pharmaceutically salt thereof which is obtainable by any of the above processes.

The term "obtainable" and "obtained" have the same meaning and are used interchangeably. In any case, the expression "obtainable" encompasses the term "obtained".

### Uses of the polymers, nanoparticles and compositions of the invention

In one aspect the present invention provides the use of the polymers as coating agents.

The polymer of the invention can be applied on so non-related surfaces such as the one of an adenoviral vector, such as a AAV, or on the surface of a device, for example a stent. In any case, the polymer of the invention will provide the surprising anti-fouling and safety properties.

The coating can be based on the immobilization of the polymer chains of formula (I) due to covalent, electrostatic or hydrogen bonds.

In the case of using the polymers of the invention to coat a AAV surface, the skilled person could routinary find the more appropriate conditions to react and bind the polymers to the amino groups of viral capsid proteins.

The polymers of formula (I), and of formula (Iter), the latter if present, can be non-covalently bound to the viral vector. There are well-known protocols to achieve such kind of binding. For example, EP3406265 provides reactions conditions which can also be followed in the context of the invention.

Alternatively, the polymer(s) of formula (I), and of formula (Iter), the latter if present, can be covalently bound to the viral vector. In this embodiment, the polymer of the invention is firstly carboxyl-activated and, then it is subjected to reaction with the viral vector under conditions promoting the reaction between the carboxylic acid of the polymer and the amino group of the viral capsid, to form an amide group.

As used herein, the term "carboxyl-activated" refers to the incorporation of a carboxyl group in which one hydroxyl group has been replaced by a carboxyl activating group. There are well-known protocols to perform such modification.

As used herein, the term "carboxyl activating group" means a group that modifies a carboxyl group to be susceptible to react later on, for instance, to form an amide group with an amino group of a viral capsid proteins. Commonly, a carboxyl activating group is an electron withdrawing moiety that substitutes the hydroxyl moiety of a carboxyl group. Such electron withdrawing moiety enhances polarization and thereby the electrophilicity at the carbonyl carbon.

In particular, the AAV vector and the acid activated PBAE as defined herein above may be mixed in solution of Hepes from 10 mM to 40 mM, such as 20 mM, and a pH from 7 to 9 such as of 7.4 at concentrations appropriate to obtain the desired ratio AAV vector/acid activated PBAE and to enable the reaction between the acid activated residues of the PBAEs and the amino groups of the viral capsid proteins and, consequently, to obtain a coating covalently attached to the AAV vector. Thus, to form the AAV vector particles a ratio from 1E-9 to 5E-8 µg pBAE/vp, for example of 1E-8 µg pBAE/vp, is used.

A PBAE-covalently coated AAV vector obtainable by the process as defined above also forms part of the invention.

The viral particle can be any vector suitable for use in therapy, particularly an AAV.

### Examples

### I. MATERIALS

5-amino-1-pentanol (ref:123048, Merck), hexylamine (ref:8.04326, Merck), 1,4-butanediol (ref:493732, Merck), 4-Cyano-4-(dodecylsulfanyl thiocarbonyl) sulfanylpentanoic acid (ref: 723274, Merck), 4- Cyano- 4- (phenylcarbonothioylthio) pentanoic acid (ref: 722995, Merck), N,N'-Dicyclohexylcarbodiimide, (ref: 36650, Merck), 4-(Dimethylamino)pyridine (ref: 8,51055, Merck), ([2-(Methacryloyloxy)ethyl]dimethyl-(3-sulfopropyl)ammonium hydroxide (ref: 494158, FluoroChem). Cys-His-His-His and Cys-Lys-Lys-Lys were ordered to Ontores. pDNA for polyplex formulation was amplified by using a Nucleobond PC 10000 EF kit (ref: 740548,Cultek). Sodium Acetate buffer was purchased at Merck (ref:S7899) and diluted to appropriate concentration using MilliQ water.

Cell Culture: AML-12 (ATCC CRL-2254) cells were cultured in DMEM-F12 supplemented with 10% (v/v) FBS, 100 units mL-1 penicillin G, 100 µg mL-1 streptomycin, and 2 mmol L-1 I-glutamine. AML-12 culture media, also included 10 µg mL-1 insulin, 5.5 µg mL-1 transferrin, 5 ng mL-1 selenium, and 40 ng mL-1 dexamethasone. All were cultured at 37oC under a 5% CO2/95% air atmosphere until 80-90% confluence before passaging or starting transfection.

### II. METHODS

1H-RMN was performed using a 400 MHz Varian (NMR Instruments, Claredon Hills, IL) IR was performed using FT-IR Nicolet iZ10 (Thermoscientific)..

The mean diameter of the polyplexes were determined using Zetasizer ZS Nano from Malvern Instruments (UK). The dynamic light scattering was measured at a scattering angle of 173° and at a temperature of 25 ° C, using a sample diluted with MilliQ water. UV reading of electrophoretic gels was performed using Tecan Infinite 200 Pro plate reader.

Flow cytometry (NovoCyte, ACEA Bioscience) was used to quantify uptake efficiency. Briefly, at the end of the experiment, cells were trypsinized and fixed with 1% paraformaldehyde. At least 2000 cells were analyzed for each well. Results correspond to the mean standard deviation of at least three independent experiments, each consisting on an experiment triplicate.

### III. SYNTHESIS OF THE POLYMERS OF THE INVENTION

### Example 1. Synthesis of poly(beta-aminoester) C6-50 backbone (pBAE)

Synthesis of the pBAE backbone was performed according following the procedure of US20180250410. Briefly, it was performed the addition reaction of the hexylamine (0.422 g) to 1,4-butanediol diacrylate (2.0 g) and 5-amino-1-pentanol (0.426 g) under magnetic stirring at 200 rpm, at a temperature of 90 °C for 20 h. The resulting polymer was characterized by 1H-RMN:(400MHz, chloroform-*d*, TMS) (ppm): δ =6,40 (d, CH₂=CH-), 6,10 (d, CH₂=CH-), 5,83 (d, CH₂=CH-), 4.18 (br, CH₂-O-C(=O)-CH=CH₂), 4,09 (t, -CH₂-CH₂-O-), 3.62 (t, CH₂-CH₂-OH), 2.78 (br, -CH₂-CH₂-N-), 2.45 (br, -N-CH₂-CH₂-C(=O)-O), 1.83 - 1.60 (br, -O-CH₂-CH₂-CH₂-CH₂-O), 1.40- 1.18 (br, - CH₂-CH2-CH₂-CH₂-OH, N-(CH₂)₂-CH₂-(CH₂)₂-OH), 0.88 (t, CH₂-CH₂-CH₃).

### Example 2. Synthesis of the chain transfer agent-modified poly(beta-aminoester) C6-50 (CTA-pBAE)

The CTA-poly(beta-aminoester) was synthesized by esterification of the hydroxyl group of the side chain of the pBAE obtained in Example 1 with the terminal acid of a CTA selected from 4-cyano-4-(dodecylsulfanyl thiocarbonyl)sulfanylpentanoic acid (DCTP) and 4-cyano-4- (phenylcarbonothioylthio) pentanoic acid. Briefly, pBAE (0.1 g) was dissolved in

anhydrous dichloromethane (2.5 mL), and dicyclohexylcarbodiimide (DCC, 0.96 g), 4-dimethylaminopyridine (DMAP, 0.005 g) and the corresponding CTA (0.096 g) were added while keeping the reaction on ice. After 15 min, the set up was let at room temperature for 18h under stirring. Molar ratio of hydroxyl:CTA 1:1 ensure 100% of esterification of hydroxyl groups from the initial backbone.

**TABLE 1**

| Example no. | CTA | Amount of CTA (g) | 1H-RMN |
|---|---|---|---|
| Ex. 2.1 | 4-cyano-4-(dodecylsulfanyl thiocarbonyl) sulfanylpentanoic acid (DCTP) | 0.066 g | ¹H NMR (400 MHz, cdcl₃, TMS) (ppm): δ =6.40 (d, CH₂=CH-), 6.10 (d, CH₂=CH-), 5.83 (d, CH₂=CH-), 4.18 (br, CH2-O-C(=O)-CH=CH₂), 4.09 (t, -CH₂-C_{H2}-O-), 3.62 (t, CH₂-CH₂-OH), 3.31 (t, -CH₂-CH₂-COO )-, 2.78 (br, -CH₂-CH₂-N-), 2.45 (br, -N-CH₂-CH₂-C(=O)- O), 1.83 - 1.60 (br, -O-CH₂-CH₂-CH₂-CH2-O), 1.40- 1.18 (br, - CH₂-CH2-CH₂-CH₂-OH, N-(CH₂)2-CH2-(CH₂)₂-OH), 0.88 (t, CH₂-CH₂-CH3) |
| Ex. 2.2 | 4-cyano- 4-(phenylcarbonothioylthio) pentanoic acid (CTP) | 0.096 g | ¹H NMR (400 MHz, cdcl₃, TMS) (ppm): δ =7.52-6.98 (m, benzene), 6.40 (d, CH₂=CH-), 6.10 (d, CH₂=CH-), 5.83 (d, CH₂=CH-), 4.18 (br, CH₂-O-C(=O)-CH=CH₂), 4.09 (t, -CH₂-CH₂-O-), 3.62 (t, CH₂-CH₂-OH), 2.78 (br, -CH₂-CH₂-N-), 2.45 (br, -N-CH₂-CH₂-C(=O)- O), 1.91 (t, -CH₃), 1.83 - 1.60 (br, -O-CH₂-CH₂-CH₂-CH₂-O), 1.40- 1.18 (br, - CH₂-CH2-CH₂-CH₂-OH, N-(CH₂)2-CH2-(CH₂)₂-OH), 0.88 (t, CH₂-CH2-CH3) |

### Example 3 Synthesis of the oligopeptide modified CTA poly(beta-aminoester) (OM-CTA-pBAE)

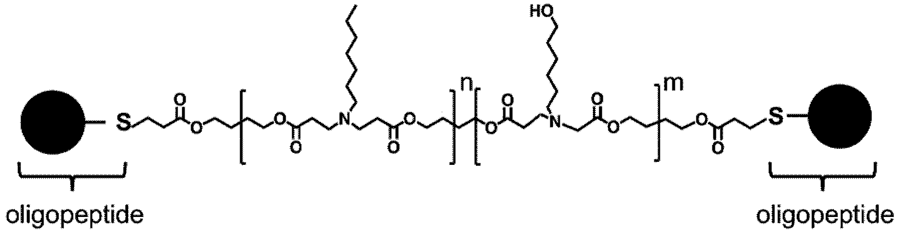

OM-CTA-pBAEs were obtained by end-modification of the acrylate-terminated groups of either pBAE (as disclosed in Ex. 1) or CTA-pBAE (as disclosed in Ex. 2), with thiol-terminated oligopeptides (comprising lysine (K) or histidine (H) residues) at a 1 : 2.5 molar ratio in dimethylsulfoxide. The mixture was stirred overnight at 200 rpm, at room temperature, and the resulting polymer was obtained by precipitation in a mixture of diethyl ether and acetone (7 : 3; v/v).

Following this procedure, the following oligopeptide-modified pBAEs were obtained:

**TABLE 2**

| OM-CTA-pBAE Ex. no. | pBAE Ex. no. | Oligopeptide (SEQ ID NO) | 1 H-RMN characterization |
|---|---|---|---|
| Ex. 3.1 | Ex. 2.1 | -CKKK (SEQ ID NO: 1) | ¹H NMR (400 MHz, cdcl₃, TMS) (ppm): δ =4.18 (br, CH₂-O-C(=O)-CH=CH₂), 4.09 (t, -CH₂-CH₂-O-), 3.62 (t, CH₂-CH₂-OH), 3.31 (t, -CH₂-CH₂-COO )-2.78 (br, -CH₂-CH₂-N-), 2.45 (br, -N-CH₂-CH₂-C(=O)- O), 1.83 - 1.60 (br, - O-CH2-CH2-CH2-CH2-O), 1.40- 1.18 (br, - CH₂-CH2-CH2-CH2-OH, N-(CH2)2-CH2-(CH2)2-OH), 0.88 (t, CH2-CH2-CH3) |
| Ex. 3.2 | Ex.2.2 | -CKKK (SEQ ID NO: 1) | ¹H NMR (400 MHz, cdcl₃, TMS) (ppm): δ =7.52-6.98 (m, benzene), 4.18 (br, CH₂-O-C(=O)-CH=CH₂), 4.09 (t, -CH₂-CH2-O-), 3.62 (t, CH₂-CH₂-OH), 2.78 (br, -CH₂-CH₂-N-), 2.45 (br, -N-CH₂-CH₂-C(=O)- O), 1.91 (t, -CH₃), 1.83 - 1.60 (br, -O-CH₂-CH2-CH2-CH2-O), 1.40-1.18 (br, - CH₂-CH2-CH₂-CH2-OH, N-(CH2)2-CH2-(CH2)2-OH), 0.88 (t, CH₂-CH2-CH3) |

| OM-pBAE Ex. no. | pBAE Ex. no. | Oligopeptide (SEQ ID NO) | 1 H-RMN characterization |
|---|---|---|---|
| Ex. 3.3 | Ex 1 | -CHHH (SEQ ID NO: 2) | ¹H-NMR (400MHz, Chloroform-d, TMS) (ppm): δ = 4.18 (br, CH₂-O-C(=O)-CH=CH₂), 4.09 (t, -CH₂-C_{H2}-O-), 3.62 (t, CH₂-CH₂-OH), 2.78 (br, -CH₂-CH₂-N-), 2.45 (br, -N-CH₂-CH₂-C(=O)- O), 1.83 - 1.60 (br, -O-CH₂-CH₂-CH₂-CH₂-O), 1.40- 1.18 (br, - CH₂-CH2-CH₂-CH₂-OH, N-(CH₂)₂-CH2-(CH2)2-OH), 0.88 (t, CH₂-CH2-CH3) |
| Ex. 3.4 | Ex 1 | -CKKK (SEQ ID NO: 1) | ¹H-NMR (400MHz, Chloroform-d, TMS) (ppm): δ = 4.18 (br, CH₂-O-C(=O)-CH=CH₂), 4.09 (t, -CH₂-C_{H2}-O-), 3.62 (t, CH₂-CH₂-OH), 2.78 (br, -CH₂-CH₂-N-), 2.45 (br, -N-CH₂-CH₂-C(=O)- O), 1.83 - 1.60 (br, -O-CH₂-CH₂-CH₂-CH₂-O), 1.40- 1.18 (br, - CH₂-CH2-CH₂-CH₂-OH, N-(CH₂)₂-CH2-(CH2)2-OH), 0.88 (t, CH₂-CH2-CH3) |

### Example 4. RAFT polymerization grafting for zwitterionic poly(beta-aminoester) obtention (OM-PZ-pBAE)

For 25-monomer length zwitterionic chains, the corresponding OM-CTA-pBAE (0.1 g) and sulfobetaine methacrylate (SBMA, 0.423 g) were dissolved in trifluoroethanol (TFE) and bubbled with N₂ for 15 min. The reaction was heated until 50 °C and V044 (0.005 g) was added as initiator. The reaction was stirred for 18h at 50 °C and under nitrogen atmosphere. The product was precipitated in diethyl ether/acetone 7:3, washed with cold methanol, and dried over vacuum to obtain the desired OM-pZ-pBAE as a yellowish powder. For 50-monomer length zwitterionic chains, same conditions were applied but 0.843g of SBMA were initially added.

Following the above procedure, the following zwitterionic pBAE polymers were obtained and characterized:

**TABLE 3**

| OM-PZ-pBAE Ex. no. | Monomer | Monomer (9) | PZ length (number of monomers | OM-CTA-pBAE | 1H-RMN characterization | IR |
|---|---|---|---|---|---|---|
| Ex. 4.1 | SBMA | 0.423 g | 25 | Ex. 3.1 | ¹H NMR (400 MHz, d₂O TMS) (ppm) δ= 4.40 (s, -COO-CH₂-), 3.71 (d, -CH₂-N-), 3.50 (d, -N-CH₂-), 3.13 (s, -CH₃), 2.88 (t, -CH₂-CH₂-SO₃), 2.17(br, -CH₃,-CH₂-CH₂-CH₂-), 1.90 (t, -CH₂-CH₂-SO₃), 1.83 - 1.60 (br, -O-CH₂-CH₂-CH₂-CH₂-O), 1.40- 1.18 (br,-CH₂-CH2-CH₂-CH₂-OH, N-(CH₂)₂-CH₂-(CH₂)₂-OH), 0.88 (t, CH₂-CH2-CH3). | 1718 (-C=O), 1644 (-C-N+), 1029 (-S=O). |
| Ex. 4.2 | SBMA | 0.846 g | 50 | Ex. 3.1 | ¹H NMR (400 MHz, d₂O TMS) (ppm) δ= 4.40 (s, -COO-CH₂-), 3.71 (d, -CH₂-N-), 3.50 (d, -N-CH₂-), 3.13 (s, -CH₃), 2.88 (t, -CH₂-CH₂-SO₃), 2.17(br, -CH₃, - CH₂-CH₂-CH₂-), 1.90 (t, -CH₂-CH₂-SO₃), 1.83 - 1.60 (br, -O-CH₂-CH₂-CH₂-CH₂-O), 1.40- 1.18 (br,-CH₂-CH2-CH₂-CH₂-OH, N-(CH₂)₂-CH₂-(CH₂)₂-OH), 0.88 (t, CH₂-CH₂-CH3). | |
| Ex. 4.3 | SBMA | 0.423 g | 25 | Ex. 3.2 | ¹H NMR (400 MHz, d₂O TMS) (ppm) δ= 4.40 (s, -COO-CH₂-), 3.71 (d, -CH₂-N-), 3.50 (d, -N-CH₂-), 3.13 (s, -CH₃), 2.88 (t, -CH₂-CH₂-SO₃), 2.17(br, -CH₃, - CH₂-CH₂-CH₂-), 1.90 (t, -CH₂-CH₂-SO₃), 1.83 - 1.60 (br, -O-CH₂-CH₂-CH₂-CH₂-O), 1.40- 1.18 (br, - CH₂-CH2-CH₂-CH₂-OH, N-(CH₂)₂-CH₂-(CH₂)₂-OH), 0.88 (t, CH₂-CH₂-CH3). | |
| Ex. 4.4 | SBMA | 0.846 g | 50 | Ex. 3.2 | ¹H NMR (400 MHz, d₂O TMS) (ppm) δ= 4.40 (s, -COO-CH₂-), 3.71 (d, -CH₂-N-), 3.50 (d, -N-CH₂-), 3.13 (s, -CH₃), 2.88 (t, -CH₂-CH₂-SO₃), 2.17(br, -CH₃,-CH₂-CH₂-CH₂-), 1.90 (t, -CH₂-CH₂-SO₃), 1.83 - 1.60 (br, -O-CH₂-CH₂-CH₂-CH₂-O), 1.40- 1.18 (br, - CH₂-CH2-CH₂-CH₂-OH, N-(CH₂)₂-CH₂-(CH₂)₂-OH), 0.88 (t, CH₂-CH₂-CH3). | |

### IV. SYNTHESIS OF DNA POLYPLEXES AND BIOPHYSICAL CHARACTERIZATION

### Example 5: DNA polyplexes with a OM-PZ-pBAE of the invention

Nanoparticles were formulated by mixing the corresponding OM-PZ-pBAE resulting from Example 4 with plasmidic DNA.

The plasmidic DNA was obtained using a GigaPrep kit and following manufacturer's instructions. Briefly, 2L media for growing the bacteria was prepared (LB media) and autoclaved. Next, a plaque was seeded with bacteria previously transformed with the required plasmid and kept in glycerols. Following, a preculture, followed by a culture of bateria were prepared. When the optical density was significantly increased, bacteria were centrifuged and the DNA extracted from the pellet. Finally, a solution of 1mg/mL of plasmid DNA was obtained.

On the other hand, 100 mg of each one of the OM-PZ-pBAE were dissolved in 1 mL of milliQ water.

Polyplexes were formed by mixing equal volumes of polymer and plasmidic DNA, with acetate buffer at 12.5 mM (most convenient formulation although it can be increased up to 25 mM), 5.2 pH adjusted by adding acetic acid 1 M. The plasmidic DNA was added to the polymer solution, mixed with pipetting for a few seconds and incubated at room temperature for 20 min (accepted range from 5 minutes to 1 hour). Finally, polyplexes were precipitated in the same volume of water.

Several formulations were prepared on the basis of different weight ratio polymer:plasmidic DNA, from 10:1 and 300:1 as provided in the table below.

**TABLE 4**

| *Polyplex no.* | *OM-pZ-pBAE* | *OM-PZ-pBAE:pDNA (w*/*w)* |
|---|---|---|
| *Ex. 5.1* | *Ex. 4.1* | *300:1* |
| *Ex. 5.2* | | *200:1* |
| *Ex. 5.3* | | *100:1* |
| *Ex. 5.4* | | *80:1* |
| *Ex. 5.5* | | *70:1* |
| *Ex. 5.6* | | *60:1* |
| *Ex. 5.7* | | *50:1* |
| *Ex. 5.8* | | *40:1* |
| *Ex. 5.9* | | *30:1* |
| *Ex. 5.10* | | *20:1* |
| *Ex. 5.11* | | *10:1* |
| *Ex. 5.12* | *Ex. 4.2* | *300:1* |
| *Ex. 5.13* | | *200:1* |
| *Ex. 5.14* | | *100:1* |
| *Ex. 5.15* | | *80:1* |
| *Ex. 5.16* | | 70:1 |
| *Ex. 5.17* | | *60:1* |
| *Ex. 5.18* | | *50:1* |
| *Ex. 5.19* | | *40:1* |
| *Ex. 5.20* | | *30:1* |
| *Ex. 5.21* | | *20:1* |
| *Ex. 5.22* | | *10:1* |
| *Ex. 5.23* | *Ex. 4.3* | *70:1* |
| *Ex. 5.24* | *Ex. 4.4* | *70:1* |

### Example 6: DNA polyplex combination comprising OM-PZ-pBAEs of the invention

As nanoparticles are internalized in endosomes by cells, it has been postulated that endosome lysis is necessary to produce gene release and cell transfection. For this reason, once the optimal ratio of zwitterionic modified pBAE and plasmid was obtained, each one of the lysine modified zwitterionic pBAEs of Examples 4.1 to 4.4 was mixed with histidine modified pBAE of Ex. 3.3, in order to ease the endosomal scape through the proton sponge effect.

The preparation protocol was the same as the one disclosed in Example 5 above with the following minor changes: separate solutions were prepared dissolving 0.1 g of the OM-PZ-pBAE of Examples 4.1 to 4.4 or of the one of Ex. 3.3, dissolved in 1 mL of miliQ water and DMSO respectively, and the resulting two solutions were mixed at different volume ratios, as summarized in Table 5a-5c below, to optimize the physicochemical properties:

**TABLE 5a**

| *Combination Ex. No.* | *Name* | *OM-pZ-pBAE***:OM-pBAE** (w*/*w)* |
|---|---|---|
| *6.1* | *C6-DCTP-K-PSBMA25 90:10* | *90:10* |
| *6.2* | *C6-DCTP-K-PSBMA25 80:20* | *80:20* |
| *6.3* | *C6-DCTP-K-PSBMA25 70:30* | *70:30* |
| *6.4* | *C6-DCTP-K-PSBMA25 60:40* | *60:40* |
| *6.5* | *C6-DCTP-K-PSBMA25 50:50* | *50:50* |
| *6.6* | *C6-DCTP-K-PSBMA25 40:60* | *40:60* |
| *6.7* | *C6-DCTP-K-PSBMA25 30:70* | *30:70* |
| *6.8* | *C6-DCTP-K-PSBMA25 20:80* | *20:80* |
| *6.9* | *C6-DCTP-K-PSBMA25 10:90* | *10:90* |

| | | |
|---|---|---|
| OM-PZ-pBAE*= Example 4.1; OM-pBAE**= Example 3.3 | | |

**TABLE 5b**

| *Ex. No.* | *Name* | *OM-PZ-pBAE*:OM-pBAE** (w*/*w)* |
|---|---|---|
| *6.10* | *C6-DCTP-K-PSBMA50 90:10* | *90:10* |
| *6.11* | *C6-DCTP-K-PSBMA50 80:20* | *80:20* |
| *6.12* | *C6-DCTP-K-PSBMA50 70:30* | *70:30* |
| *6.13* | *C6-DCTP-K-PSBMA50 60:40* | *60:40* |
| *6.14* | *C6-DCTP-K-PSBMA50 50:50* | *50:50* |
| *6.15* | *C6-DCTP-K-PSBMA50 40:60* | *40:60* |
| *6.16* | *C6-DCTP-K-PSBMA50 30:70* | *30:70* |
| *6.17* | *C6-DCTP-K-PSBMA50 20:80* | *20:80* |
| *6.18* | *C6-DCTP-K-PSBMA50 10:90* | *10:90* |

| | | |
|---|---|---|
| OM-PZ-pBAE*= Example 4.2; OM- pBAE**= Example 3.3 | | |

**TABLE 5c**

| *Ex. No.* | *Name* | *OM-PZ-pBAE* | *OM-PZ-pBAE: OM-pBAE* (w*/*w)* |
|---|---|---|---|
| *6.19* | *C6-CTP-K-PSBMA25 30:70* | *Ex. 4.3* | *30:70* |
| *6.20* | *C6-CTP-K-PSBMA50 80:20* | *Ex. 4.4* | *30:70* |

| | | | |
|---|---|---|---|
| OM-pBAE*= Example 3.3 | | | |

The resulting solutions were then mixed with the plasmid and the same reaction conditions as the ones disclosed in Example 5 were followed to obtain the polyplexes of the invention.

### Example 7. DNA polyplexes with pBAE of Ex. 3. (comparative purpose)

In order to determine the effect of covalently binding the zwitterionic polymer to the pBAE backbone, a comparative test was performed using OM-pBAEs of Example 3 (which do not incorporate neither the zwitterionic polymer nor the chain transfer agent) to form the DNA polyplexes.

The same protocol as the one provided under Example 5 was followed in order to prepare a solution for each one of the OM-pBAE, the histidine-modified pBAE (Ex. 3.3, also referred as "H" solution) and the lysine-modified pBAE (Ex. 3.4, also referred as "K" solution).

Then, both solutions were mixed at a K solution:H solution weight ratio of 60:40, in acetate buffer at 12.5 mM, 5.2 pH, adjusted by adding acetic acid 1 M. Thus, obtaining a polymeric solution K-H.

The polyplexes were formed by mixing equal volumes of polymeric solution K-H and the plasmid solution (the latter being obtained as explained in previous sections, but at different weight ratio polymer:genetic material, from 10:1 and 300:1. The genetic material was added to polymer solution, mixed with pipetting for a few seconds and incubated at room temperature for 20 min (accepted range from 5 minutes to 1 hour). Finally, polyplexes were precipitated in the same volume of water.

### Example 8. DNA polyplexes with a pBAE coated with a zwitterionic polymer (comparative purpose)

In order to determine how the interaction between the pBAE and the zwitterionic polymer affects to the properties of the resulting DNA polyplexes, a comparative one, based on electrostatic interactions (instead of covalent interaction) was prepared as follows.

The same protocol as the one provided under Example 5 was followed in order to prepare a solution for each one of the OM-pBAE, the histidine-modified pBAE (Ex. 3.3, also referred as "H" solution) and the lysine-modified pBAE (Ex. 3.4, also referred as "K" solution).

Then, both solutions were mixed at a K solution:H solution weight ratio of 60:40, in acetate buffer at 12.5 mM, 5.2 pH, adjusted by adding acetic acid 1 M. Thus, obtaining a polymeric solution K-H.

The polyplexes were formed by mixing equal volumes of polymeric solution K-H and the plasmid solution (the latter being obtained as explained in previous sections) but at different weight ratio polymer:genetic material, from 10:1 and 300:1. The genetic material was added to polymer solution, mixed with pipetting for a few seconds and incubated at room temperature for 20 min (accepted range from 5 minutes to 1 hour). After 10 minutes, different percentages of pSBMA (chain length 25 monomers) were added into the acetate buffer medium and gently resuspended. After 10 minutes, polyplexes were precipitated in the same volume of water.

**TABLE 6**

| *Poliplex Ex.No* | *Percentage of pSBMA (%w pSBMA*/*w pBAE)* |
|---|---|
| *8.1* | *0.4* |
| *8.2* | *0.04* |
| *8.3* | *0.004* |

### Example 9. Biophysical characterization of the polyplexes

### A) Encapsulation efficiency

In order to confirm the encapsulation efficiency of the polyplexes obtained as disclosed in the above Examples 5 to 8, an agarose gel electrophoresis was performed. Briefly, 8 uL of the polyplexes resulting from Examples 5-8 were added to wells of agarose gel (2.5%, containing 1 µg ml⁻¹ ethidium bromide). Samples were run at 80 V for 45 min and visualized by UV illumination.

In order to determine the efficiency from the electrophoretic data, bands were quantified using ImageJ software. The naked plasmid was used as control, to normalize the intensity of the bands corresponding to the encapsulated plasmid and determine the percentage that has not been encapsulated (the one that runs in the gel). Then, by difference, the percentage encapsulated was calculated.

In this way, it was found that the amount of polymer guaranteeing the encapsulation of the DNA sample n the case of using the polymers of the invention, was the same as for pBAE alone, (Ex. 7) i.e., 20:1. This means that the covalent binding of the zwitterionic polymeric chains does not negatively affect the "innate" encapsulation ability of pBAE.

On the other hand, the inventors found that the encapsulation efficiency was not neither negatively affected by the length of the zwitterionic polymer. To that end, they compared the results from Ex. 5.23 (C6CTPKPSBMA50), having a zwitterionic polymer with 50 SBMA monomers, vs Ex. 5.24 (C6CTPKPSBMA25), having a zwitterionic polymer with 25 SBMA monomers: both were able to substantially encapsulate the same amount of nucleic acid.

### B) Size analysis

Polyplexes were synthesized as disclosed in previous examples. After 20 min of incubation at room temperature, 100 µl of nanoparticles were diluted with 900 µl of PBS 1× for further hydrodynamic size analysis.

On one hand, it was found that the polyplexes of the invention had low average size, remarkably lower than 1000 nm, as it is shown in Table 7 below, make them suitable to be used as delivery vehicles:

**Table 7**

| Polyplex Ex. no. | Average size (nm) | Stand. Dev. (nm) |
|---|---|---|
| Ex. 5.4 | 365.6 | 48.2 |
| Ex. 5.5 | 343.8 | 40.2 |
| Ex. 5.6 | 645.3 | 67.2 |
| Ex. 5.7 | 846.7 | 52.6 |
| Ex. 5.8 | 454.4 | 45.3 |
| Ex. 5.9 | 595.7 | 46.0 |
| Ex. 5.10 | 562.2 | 111.8 |
| Ex. 5.11 | 365.6 | 9.4 |
| Ex. 5.12 | 648.3 | 29.9 |
| Ex. 5.16 | 84.5 | 5.2 |
| Ex. 5.17 | 425.8 | 77.4 |
| Ex. 5.21 | 165.5 | 41.7 |
| Ex. 5.22 | 261.4 | 41.7 |

In view of the above, the inventors confirmed the effect of adding a second polymer, OM-pBAE to the polyplexe. Thus, the inventors compared the polyplexes of Example 5.5 with those of Example 6, formulated at the same weight ratio of pBAE polymers:pDNA. The results are summarized in Table 8 below:

**Table 8**

| Polyplex Ex. no. | *OM-PZ-pBAE:pDNA* (w/w) | Average size (nm) | Stand. Dev. (nm) |
|---|---|---|---|
| Ex. 6.2 | 70:1 | 295.1 | 6.9 |
| Ex. 6.3 | 70:1 | 256.5 | 19.0 |
| Ex. 5.4 | 70:1 | 312.0 | 5.8 |
| Ex. 6.5 | 70:1 | 341.9 | 15.6 |
| Ex. 6.6 | 70:1 | 304.7 | 3.2 |
| Ex. 6.7 | 70:1 | 274.0 | 34.4 |
| Ex. 6.8 | 70:1 | 320.6 | 4.7 |
| Ex. 6.11 | 70:1 | 216.0 | 39.4 |
| Ex. 6.12 | 70:1 | 162.2 | 8.8 |
| Ex. 6.13 | 70:1 | 180.8 | 21.0 |
| Ex. 6.14 | 70:1 | 174.8 | 2.9 |
| Ex. 6.15 | 70:1 | 193.0 | 9.3 |
| Ex. 6.16 | 70:1 | 179.4 | 1.7 |
| Ex. 6.17 | 70:1 | 211.7 | 17.3 |
| Ex. 6.18 | 70:1 | 214.8 | 17.6 |
| Ex. 6.19 | 70:1 | 214.2 | 6.0 |
| Ex. 6.20 | 70:1 | 282 | 10 |
| Ex. 7 | 25:1 | 209.4 | 2.9 |
| Ex. 8.1 | 25:1 | 194.6 | 4.4 |
| Ex. 8.2 | 25:1 | 336.9 | 6.7 |
| Ex. 8.3 | 25:1 | 314.8 | 72.3 |

As it can be derived from Table 8 above, the incorporation of a second pBAE polymer, in particular a OM-pBAE polymer, further reduced the average size of the resulting polyplexes.

This is of importance because a size below 900 nm guarantees that the polyplexes can provide the biologically effect: (a) it is minimized the formation of aggregates, and (b) it is facilitated cell internalization. In addition, small sizes guarantees an appropriate stability when formulated in the form of a pharmaceutical composition and can be easily reproduced.

### C) Anti-fouling properties

Protein coronas were formed following a modification of the Sempf protocol (Sempf K. et al., "Adsorption of plasma proteins on uncoated PLGA nanoparticles", European Journal of Pharmaceutics and Biopharmaceutics, vol. 85(1), 2013, pages 53-60). Briefly, 100 µL of fresh polyplexes obtained as disclosed in previous Examples 5 to 8 were incubated with 185 µL of FBS plus 1 mL phosphate buffered saline (PBS), to simulate blood conditions, for 18 h, at 37 °C. After this time, they were centrifuged for 15 min at 15 000 × *g*, followed by the addition of 100 µL of PBS obtaining a first supernatant (S1) with the unbound protein and a first pellet (including the nanoparticles), the latter being redispersed and then recentrifuged at same conditions. In this way, a second supernatant (S2) including the soft corona was obtained. The second pellet (hard corona pellet) was redispersed with 100 µL of PBS + 10 wt% SDS, and incubated for 5 min, at 37 °C, to separate hard protein corona in a third supernatant (S3). Finally, protein concentration was determined by using a colorimetric BCA kit, following manufacturer's instructions.

The following table summarizes the protein amount determined for the tested polyplexes:

**Table 9**

| Polyplex Ex. number | Protein Corona (%) | Stand. Dev.soft corona |
|---|---|---|
| Ex. 6.3 (Invention) | 3.6 | 1.2 |
| Ex. 7 (comparative purpose) | 15.9 | 1.1 |
| Ex. 8.1 (comparative purpose) | 15.7 | 2.2 |
| Ex. 8.2 (comparative purpose) | 15.5 | 0.3 |
| Ex. 8.3 (comparative purpose) | 12.7 | 0.7 |

| | | |
|---|---|---|
| Protein corona (%) = concentration of soft+hard protein corona/total concentration of protein, wherein the "total concentration of protein" is determined adding the protein concentration determined in supernatants S1+S2+S3. | | |

As it can be derived from the above, the polyplexes of the invention, due to the grafting of the zwitterionic polymer into pBAE backbone, shows a substantially lower amount of protein adhered to the surface of the polyplexes. Particularly, it can be seen that the polyplexes of the invention had a protein corona content at least 7-fold lower than those provided by the pBAE of Example 7 (which lacks the zwitterionic polymer) and those of Example 8 (which differ from the one of Ex. 6.3 in the nature of the interaction between the pBAE and the zwitterionic polymer).

As it is also concluded from the above table 9, when the polyplex was based on an electrostatic zwitterionic polymer (comparative Example 8), there were not significant differences with respect to the pBAE with no zwitterionic polymer (comparative Example 7). This means that it is not only necessary the presence of a zwitterionic polymer, but, the most important, that the zwitterionic is covalently linked (i.e. grafted) to the pBAE backbone, as it is the case of the polymers of the invention, to achieve such remarkable improvement in the anti-fouling properties.

Since there were not significant differences between the polyplex with an electrostatic zwitterionic polymer (comparative Ex. 8) vs the pBAE with no zwitterionic polymer (comparative Ex. 7), the following tests were only performed with the polyplex just comprising the pBAE with no zwitterionic polymer.

### D) Cell viability assay

HeLa cells were grown in 96 well plates at an initial seeding density of 10000 cells per well in 200 µL cell culture media (density of 5.10⁴ cells/mL). Cells were grown for 24 h, transfected with different polymer formulations at 4 ug/well DNA. 24 h post-transfection, the medium was removed, cells were washed with PBS, and complete medium supplemented with 20% MTT reagent (v/v) was added. Cells were incubated at 37 °C for 2 hours and DMSO was added to solubilize the purple crystals. Absorbance was measured at 550 nm using a microplate reader. Cell viability was expressed as a relative percentage compared with untreated cells.

**Table 10**

| Poliplex Ex. No. | Cell viability (%) | Stand. Dev. (%) |
|---|---|---|
| 7 | 8.88 | 0.54 |
| 6.3 | 53.56 | 10.81 |
| 6.12 | 44.35 | 4.47 |
| 6.19 | 14.64 | 4.62 |
| 6.20 | 12.20 | 0.86 |

As it is derived from the above table, the polyplexes of the invention, characterized by incorporating grafted zwitterionic polymers, are substantially less cytotoxic than the polyplexes obtained using just pBAE (comparative Ex. 7), at any time and even using higher concentrations of the polyplex.

Not only that, but also, the polyplexes of the invention are safer vehicles to carry high amounts of the active ingredient.

### E) Hemolysis assay

The administration of nanocarriers is usually systemic and performed through parenteral routes. Therefore, particles get in contact with blood very frequently. For this reason, proving their compatibility with red blood cells (i.e. erythrocytes) is a must before driving in vivo studies. If the nanoparticle solution is hypoosmotic, erythrocytes will explode; while hyperosmotic solution produce a shrinkage of erythrocytes. Sometimes, even when adjusting salt concentration, particles produce hemolysis, this is, the lysis of erythrocytes. Since they are full of hemoglobin, when they are broken, they release hemoglobin; therefore, we can quantify hemoglobin release, by measuring absorbance at 540 nm. The higher the hemoglobin release, the higher the hemolysis percentage.

Human blood was centrifuged twice to isolate erythrocytes and the pellet was diluted in PBS to a concentration of 8x10⁹ cell/mL. Cells were seeded in a 96 well plate and incubated for 10 min and 24 hours with the desired concentration of DNA. The samples were centrifuged at 3000 rpm for 5 minutes and the absorbance of the supernatant was measured at 540 nm to determine the amount of hemoglobin in the supernatant of the samples.

The following table summarizes the results thus obtained:

**Table 11**

| Poliplex Ex. No. | DNA (ug/well) | % haemolysis | Stand. Dev. |
|---|---|---|---|
| 7 | 0.1 | 61.6 | 0.3 |
| | 0.3 | 5.4 | 0.3 |
| | 0.6 | 3.4 | 2.1 |
| | 1 | 0.03 | 0.04 |
| 6.3 | 0.1 | 1.5 | 0.2 |
| | 0.3 | 0.3 | 0.02 |
| | 0.6 | 0.00 | 0.00 |
| | 1 | 0.00 | 0.00 |

As it is derived from the above, the polymer of the invention provides a % of haemolysis remarkable lower, especially when significant concentrations of the polyplexes are used.

This means that the grafting of zwitterionic chains in a pBAE backbone makes the resulting polymer remarkably safer for their use in therapy.

### F) Transfection efficiency

Transfection efficiency of different formulations of polyplexes was assessed in AML12 cells, from mouse liver. Zwitterionic grafted pBAE particles (OM-pZ-pBAE) were not expected to present high transfections percentages, due to their stealth properties, while retinol modified zwitterionic pBAE :pBAE particles (KzHHret) were expected to selectively transfect AML12 cells. Retinol is highly affinity for retinol binding protein (RBP), a blood circulating protein which presents receptors in the liver. Therefore, the rational of this strategy was to make nanoparticles that naturally attached to RBP; this is, that RBP forms a protein corona in the nanoparticles of the invention to direct them to correspondent liver receptor. As a negative control, glucosamine modified zwitterionic pBAE:pBAE (KzHHglu) particles were used to transfect AML12 cells as they do not present specific receptors for this moiety. Results were compared to pBAE particles, which were previously proved to have high and non-specific transfection capacity in several cell lines.

Thus, first of all the zwitterionic pBAE polymers of the invention, as well as the comparative backbone of Ex. 3 above was modified to incorporate retinol moieties as previously reported by Fornaguera C et al., 2019.

Then, the synthesis and isolation of the polyplexes were performed following the protocol already provided above.

AML12 cells were grown in 96 well plates at an initial seeding density of 10000 cells per well in 200 µL cell culture media (5.10⁴cells/mL). Cells were grown for 24 h and transfected with different polyplex formulations encapsulating green fluorescent protein plasmid. After 48h, cells were trypsinized and flow cytometry was performed.

The results are provided below in Table 12: the polyplexes incorporating the polymer of the invention, at different percentages of retinol (KzHHret) shows a clear positive trend to transfection, this trend increasing with the concentration of retinol.

**Table 12**

| Polyplex Ex.no | Polymer ratios (w:w:w) | %Transfection | SD |
|---|---|---|---|
| Ex. 7 | KH (60:40) | 41.6 | 25.0 |
| Ex.7.1 | KHret (90:10) | 47.3 | 6.6 |
| Ex.7.2 | KHret (60:40) | 59.8 | 7.5 |
| Ex.7.3 | KHret (30:70) | 63.3 | 4.9 |
| Ex. 6.3 | KzH (30:70) | 16.3 | 6.5 |
| Ex.6.3.1 | KzHHret (90:5:5) | 32.6 | 3.4 |
| Ex.6.3.2 | KzHHret (30:65:5) | 42.2 | 1.5 |
| Ex.6.3.3 | KzHHret (60:35:5) | 40.1 | 1.7 |
| Ex.6.3.4 | KzHHret (90:10) | 60.7 | 2.3 |
| Ex.6.3.5 | KzHHret (30:70) | 72.9 | 3.0 |

It is remarkable that the polymer of the invention has not transfection ability and that only when it incorporates retinol, this effect is observed. Not only that, but also, the polymer of the invention does not mask the cell-targeting moiety therein incorporated: the greater the concentration of cell-targeting moiety, the greater the transfection rate.

### Citation List

Sempf K. et al., "Adsorption of plasma proteins on uncoated PLGA nanoparticles", European Journal of Pharmaceutics and Biopharmaceutics, vol. 85(1), 2013, pages 53-60).
Hess A. et al., "Aminolysis induced functionalization of (RAFT) polymer-dithioester with thiols and disulfides", Polym. Chem., 2020,11, 7677-7684.
Boyer C. et al., "Modification of RAFT-polymers via thiol-ene reactions: A general route to functional polymers and new architectures", Polym. Chem., 2009, 47(15), 3773-3794.
Fornaguera C. et al., "In Vivo Retargeting of Poly(beta aminoester) (OM-PBAE) Nanoparticles is Influenced by Protein Corona", Advanced HealthCare Materials, 2019, 8(19), 1900849.

## Claims

1. A poly(beta-aminoester) polymer of formula (I) or a pharmaceutically acceptable salt thereof: 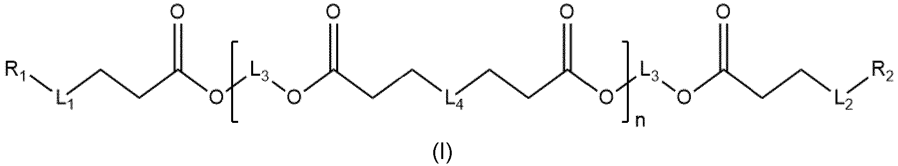 wherein
L₃ is independently selected from the group consisting of alkylene, alkenylene, heteroalkylene, heteroalkenylene, arylene, and heteroarylene; or, alternatively
at least one of L₃ is
wherein T₁ is 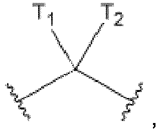 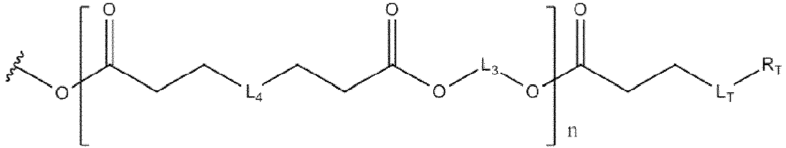
and T₂ is selected from H, alkyl, and 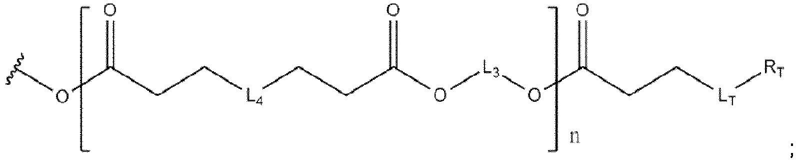
wherein L_{T} is independently selected from the group consisting of: 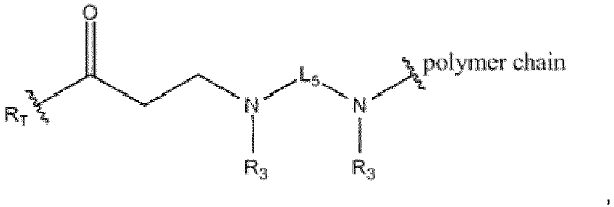
O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl,
and the remaining L₃ groups are independently selected from the group consisting of alkylene, alkenylene, heteroalkylene, heteroalkenylene, arylene, and heteroarylene;
L₄ is independently selected from the group consisting of formula (i) and (ii) 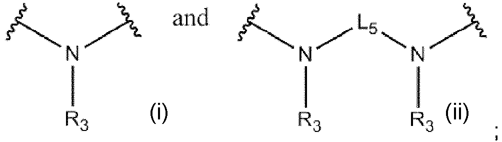
L₅ is independently selected from the group consisting of alkylene, alkenylene, heteroalkylene, heteroalkenylene, arylene, and heteroarylene;
each R₃ is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), a moiety having an amine group (-NH₂), and a moiety having a zwitterionic polymer,
wherein:
- said polyalkylene glycol is either bound directly to the nitrogen atom to which R₃ is attached or bound to the nitrogen atom to which R₃ is attached via a linker moiety, wherein said linker moiety is an alkylene, cycloalkylene, alkenylene, cycloalkenylene, heteroalkylene, heterocycloalkylene, arylene or heteroarylene group;
- the moiety having a hydroxyl group (-OH) is selected from the group consisting of -(CH₂)ₚOH, and -(CH₂)₂(OCH₂CH₂)_{q}OH;
- the moiety having an amino group (-NH₂) is selected from the group consisting of -(CH₂)ₚNH₂, and -(CH₂)₂(OCH₂CH₂)_{q}NH₂; and
- the moiety having a zwitterionic polymer is one of formula (II) 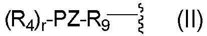
wherein R₄ represents R'₄-C(=S)-S-; R'₄ represents an aryl, heteroaryl, alkyl, -SR₅, -NR₆R₇ or -OR₈; R₅ represents aryl, heteroaryl or alkyl; R₆ and R₇ are the same or different and represent hydrogen, halogen, alkyl, cycloalkyl, alkenyl, cycloalkenyl, heteroalkyl, heterocycloalkyl, acyl, aryl, and heteroaryl; R₈ represents hydrogen, alkyl, cycloalkyl, alkenyl, cycloalkenyl, heteroalkyl, heterocycloalkyl, acyl, aryl, and heteroaryl; and R₉ is a linker which binds the PZ to the -N- of the substituent of formula (i) or (ii);
PZ represents a zwitterionic polymer comprising one or more zwitterionic monomers;
provided that at least one of the R₃ is a moiety having a zwitterionic polymer as defined above;
n is an integer from 5 to 1000, p is an integer from 1 to 20, q is an integer from 1 to 10, and r is an integer from 0 to 1;
each L₁ and L₂ is independently selected from the group consisting of: 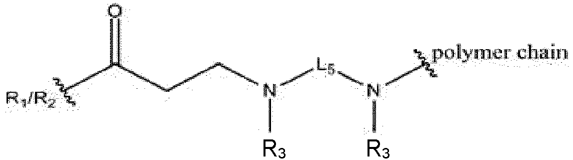
O, S, NRₓ, and a bond; wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl; and R₃ and L₅ being as defined above; and
R₁, R₂ and R_{T} are independently selected from an oligopeptide and R_{y},
R_{y} being selected from the group consisting of hydrogen, halogen, alkyl, cycloalkyl, alkenyl, cycloalkenyl, heteroalkyl, heterocycloalkyl, acyl, aryl, and heteroaryl; and
provided that at least one of R₁, R₂ and R_{T} is an oligopeptide.

2. The polymer of claim 1, wherein:
L₁ and L₂ represent a bond;
L₃ is independently selected from the group consisting of alkylene, alkenylene, heteroalkylene, heteroalkenylene, arylene, and heteroarylene; particularly L₃ represents alkylene; and
L₄ represents
wherein R₃ is as defined in claim 1.

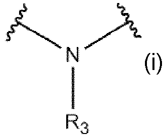

3. The polymer of any one of the preceding claims, wherein at least a 5% of the R₃ groups in the polymer of formula (I) represents a moiety having a zwitterionic polymer of formula (II) as defined in any of the above claims; particularly at least a 30%, 40%, 50%, 60%, 70%, 70%, 80% or 90% of the R₃ groups in the polymer of formula (I) represents a moiety having a zwitterionic polymer of formula (II) as defined in any of the above claims; particularly at least a 40% of the R₃ groups in the polymer of formula (I) represents a moiety having a zwitterionic polymer of formula (II) as defined in any of the above claims; particularly from 30 to 80%; particularly from 40 to 70% of the R₃ groups in the polymer of formula (I) represents a moiety having a zwitterionic polymer of formula (II) as defined in any of the above claims.

4. The polymer of any one of the preceding claims, wherein r represents 1 and the moiety having a zwitterionic polymer of formula (II) is one where R'₄ represents an aryl, alkyl or -S-R₅, wherein R₅ is as defined above; particularly, R'₄ represents an aryl, alkyl, -S-aryl or-S-alkyl.

5. The polymer of any one of the preceding claims, wherein the moiety having a zwitterionic polymer of formula (II) is one where R₉ represents -alkylene-C(O)-O-alkylene-, wherein the alkylene is optionally substituted.

6. The polymer of any one of the preceding claims, wherein the remaining R₃ groups, which are other than a moiety of formula (II), are the same or different and are selected from -(CH₂)ₚOH, -(CH₂)₂(OCH₂CH₂)_{q}OH, -(CH₂)ₚNH₂, and -(CH₂)₂(OCH₂CH₂)_{q}NH₂, wherein p and q are as defined above.

7. The polymer of the preceding claim, wherein the or each oligopeptide is of formula (III): 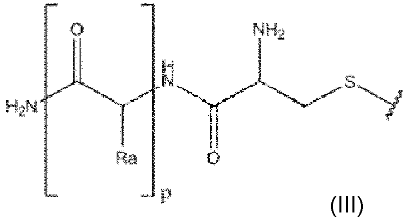 wherein p is an integer from 2 to 19 and wherein Ra is selected from the group consisting of H₂NC(=NH)-NH(CH₂)₃-, H₂N(CH₂)₄- or (1H-imidazol-4-yl)-CH₂-.

8. The polymer of any one of the preceding clams, which is functionalised with one or more cell-targeting moieties.

9. A nanoparticle, comprising one or more polymers of formula (I) as defined in any one of the preceding claims.

10. The nanoparticle of claim 9, which further comprises one or more polymers of formula (Iter): 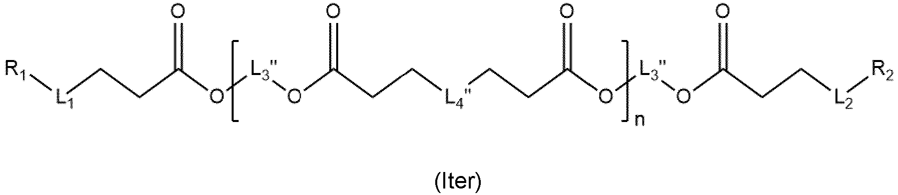
L"₃ is independently selected from the group consisting of alkylene, alkenylene, heteroalkylene, heteroalkenylene, arylene, and heteroarylene; or, alternatively
at least one of L₃" is
wherein
T₁" is 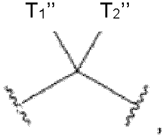 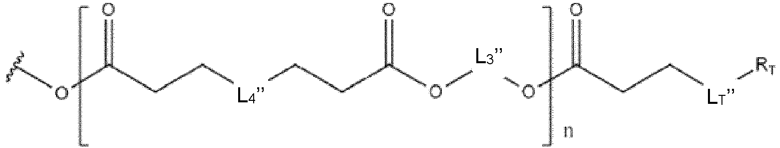
and T₂" is selected from H, alkyl, and 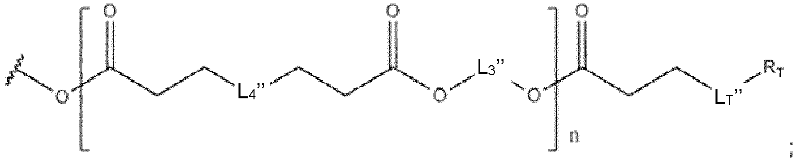
wherein L_{T}" is independently selected from the group consisting of: 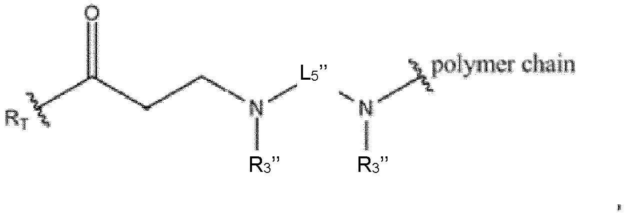
O, S, NRₓ, and a bond, wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl, and
the remaining L₃" groups are independently selected at each occurrence from the group consisting of alkylene, alkenylene, heteroalkylene, heteroalkenylene, arylene, and heteroarylene;
L"₄ is independently selected from the group consisting of (v) and (vi) 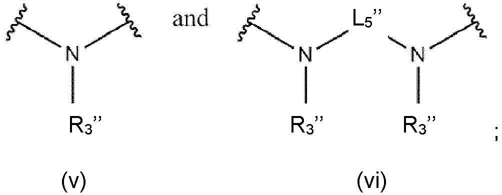
L₅" is independently selected from the group consisting of alkylene, alkenylene, heteroalkylene, heteroalkenylene, arylene, and heteroarylene;
each R₃" is independently selected from the group consisting of hydrogen, halogen, alkyl, cycloalkyl, alkenyl, cycloalkenyl, heteroalkyl, heterocycloalkyl, acyl, aryl, heteroaryl, polyalkylene glycols, a moiety having a hydroxyl group (-OH), and a moiety having an amine group (-NH₂),
wherein:
- said polyalkylene glycol is either bound directly to the nitrogen atom to which R₃" is attached or bound to the nitrogen atom to which R₃" is attached via a linker moiety, wherein said linker moiety is an alkylene, cycloalkylene, alkenylene, cycloalkenylene, heteroalkylene, heterocycloalkylene, arylene or heteroarylene group;
- the moiety having a hydroxyl group (-OH) is selected from the group consisting of - (CH₂)ₚOH, and -(CH₂)₂(OCH₂CH₂)_{q}OH; and
- the moiety having an amino group (-NH₂) is selected from the group consisting of -(CH₂)ₚNH₂, -(CH₂)ₚOH;
n is an integer from 5 to 1000, p is an integer from 1 to 20, and q is an integer from 1 to 10,
each L₁ and L₂ is independently selected from the group consisting of: 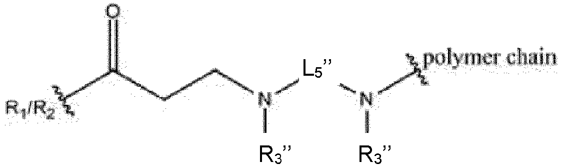
O, S, NRₓ, and a bond; wherein Rₓ is independently selected from the group consisting of hydrogen, halogen, alkyl, cycloalkyl, alkenyl, cycloalkenyl, heteroalkyl, heterocycloalkyl, acyl, aryl, and heteroaryl; and R₃" and L₅" being as defined above; and
R₁, R₂ and R_{T} are independently selected from an oligopeptide and R_{y},
R_{y} being selected from the group consisting of hydrogen, halogen, alkyl, cycloalkyl, alkenyl, cycloalkenyl, heteroalkyl, heterocycloalkyl, acyl, aryl, and heteroaryl; and provided that at least one of R₁, R₂ and R_{T} is an oligopeptide.

11. The nanoparticle of any one of the preceding claims 9-10, which comprises one or more compounds of interest, particularly selected from a diagnostic reagent, an imaging reagent and an active ingredient.

12. A pharmaceutical composition comprising a therapeutically effective amount of the nanoparticle as defined in any one of the claims 9-11, together with one or more pharmaceutically acceptable excipients.

13. A delivery system comprising an external coating comprising the polymer (I) as defined in any one of the claims 1-8 and, optionally, one or more polymers of formula (Iter) as defined in claim 10.

14. Use of the polymer of formula (I) as defined in any one of the claims 1-8, alone or in combination with one or more polymers of formula (Iter) as defined in claim 10 as a coating, particularly as anti-fouling coating; or, alternatively, as an encapsulating agent; or, alternatively, as a delivery carrier.

15. The nanoparticle as defined in any one of the claims 9-11 or the pharmaceutical composition as defined in claim 12 or the delivery system as defined in claim 13 for use in therapy.

## Patentansprüche

1. Ein Poly(beta-Aminoester)-Polymer der Formel (I) oder ein pharmazeutisch akzeptables Salz davon: 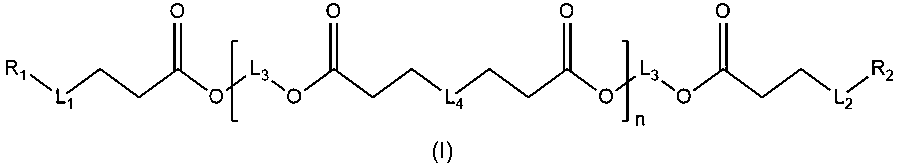 wobei
L₃ unabhängig voneinander ausgewählt ist aus der Gruppe bestehend aus Alkylen, Alkenylen, Heteroalkylen, Heteroalkenylen, Arylen und Heteroarylen; oder, ersatzweise,
mindestens einer von L₃ ist
wobei T₁ ist 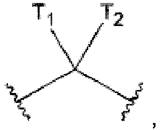 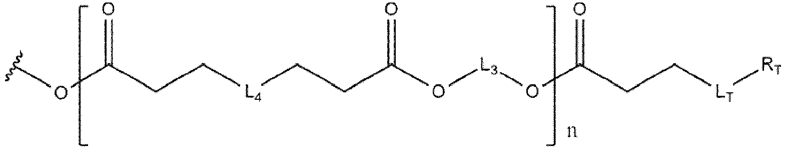
und T₂ ausgewählt ist aus H, Alkyl und 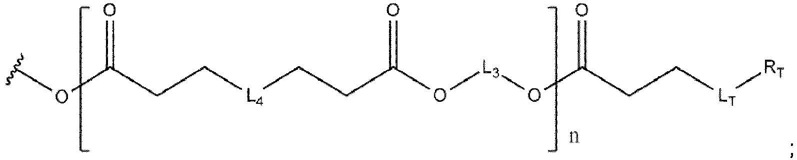
wobei L_{T} unabhängig ausgewählt ist aus der Gruppe bestehend aus: 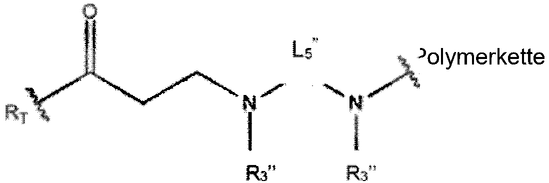
O, S, NRₓ und einer Bindung, wobei Rₓ unabhängig voneinander ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl,
und die übrigen L₃-Gruppen unabhängig voneinander ausgewählt sind aus der Gruppe bestehend aus Alkylen, Alkenylen, Heteroalkylen, Heteroalkenylen, Arylen und Heteroarylen;
L₄ unabhängig voneinander ausgewählt ist aus der Gruppe bestehend aus Formel (i) und (ii) 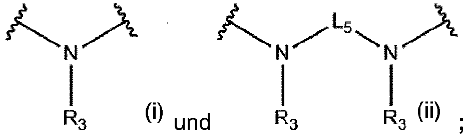
L₅ unabhängig voneinander ausgewählt ist aus der Gruppe bestehend aus Alkylen, Alkenylen, Heteroalkylen, Heteroalkenylen, Arylen und Heteroarylen;
jedes R₃ unabhängig voneinander ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl, Heteroaryl, Polyalkylenglycolen, einem Teil mit einer Hydroxylgruppe (-OH), einem Teil mit einer Amingruppe (-NH₂) und einem Teil mit einem zwitterionischen Polymer,
wobei:
- das Polyalkylenglykol entweder direkt an das Stickstoffatom gebunden ist, an welches R₃ gebunden ist, oder über einen linkenden Teil an das Stickstoffatom gebunden ist, an welches R₃ gebunden ist, wobei der linkende Teil eine Alkylen-, Cycloalkylen-, Alkenylen-, Cycloalkenylen-, Heteroalkylen-, Heterocycloalkylen-, Arylen- oder Heteroarylengruppe ist;
- der Rest mit einer Hydroxylgruppe (-OH) ausgewählt ist aus der Gruppe bestehend aus -(CH₂)ₚOH und -(CH₂)₂(OCH₂CH₂)_{q}OH;
- der Teil mit einer Aminogruppe (-NH₂) aus der Gruppe ausgewählt ist, die aus -(CH₂)ₚNH₂ und -(CH₂)₂(OCH₂CH₂)_{q}NH₂ besteht; und
- der Teil mit einem zwitterionischen Polymer einer der Formel (II) ist 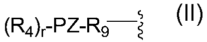
wobei R₄ für R'₄-C(=S)-S- steht; R'₄ für ein Aryl, Heteroaryl, Alkyl, -SR₅, -NR₆R₇ oder -OR₈ steht; R₅ für Aryl, Heteroaryl oder Alkyl steht; R₆ und R₇ gleich oder verschieden sind und für Wasserstoff, Halogen, Alkyl, Cycloalkyl, Alkenyl, Cycloalkenyl, Heteroalkyl, Heterocycloalkyl, Acyl, Aryl und Heteroaryl stehen; R₈ für Wasserstoff, Alkyl, Cycloalkyl, Alkenyl, Cycloalkenyl, Heteroalkyl, Heterocycloalkyl, Acyl, Aryl und Heteroaryl steht; und R₉ ein Linker ist, der das PZ an das -N- des Substituenten der Formel (i) oder (ii) bindet;
PZ für ein zwitterionisches Polymer steht, das ein oder mehrere zwitterionische Monomere umfasst;
unter der Voraussetzung, dass mindestens einer der R₃ ein Teil ist, der ein zwitterionisches Polymer wie oben definiert hat;
n eine ganze Zahl von 5 bis 1000 ist, p eine ganze Zahl von 1 bis 20 ist, q eine ganze Zahl von 1 bis 10 ist und r eine ganze Zahl von 0 bis 1 ist;
jedes L₁ und L₂ unabhängig voneinander ausgewählt ist aus der Gruppe bestehend aus: 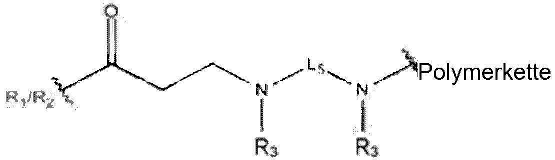
O, S, NRₓ und einer Bindung; wobei Rₓ unabhängig ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl; und R₃ und L₅ wie oben definiert sind; und
R₁, R₂ und R_{T} unabhängig voneinander aus einem Oligopeptid und R_{y} ausgewählt sind,
R_{y} ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl; und
unter der Voraussetzung, dass mindestens eines von R₁, R₂ und R_{T} ein Oligopeptid ist.

2. Das Polymer von Anspruch 1, wobei:
L₁ und L₂ für eine Bindung stehen;
L₃ unabhängig voneinander ausgewählt ist aus der Gruppe bestehend aus Alkylen, Alkenylen, Heteroalkylen, Heteroalkenylen, Arylen und Heteroarylen; insbesondere steht L₃ für Alkylen; und
L₄ steht für 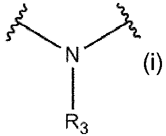
wobei R₃ wie in Anspruch 1 definiert ist.

3. Das Polymer von einem der vorhergehenden Ansprüche, wobei mindestens 5 % der R₃-Gruppen in dem Polymer von Formel (I) für einen Teil mit einem zwitterionischen Polymer von Formel (II), wie in einem der vorhergehenden Ansprüche definiert, steht; insbesondere mindestens 30 %, 40 %, 50 %, 60 %, 70 %, 70 %, 80 % oder 90 % der R₃-Gruppen in dem Polymer von Formel (I) für einen Teil mit einem zwitterionischen Polymer von Formel (II), wie in einem der vorhergehenden Ansprüche definiert, steht; insbesondere mindestens 40 % der R₃-Gruppen in dem Polymer von Formel (I) für einen Teil mit einem zwitterionischen Polymer von Formel (II), wie in einem der vorhergehenden Ansprüche definiert, steht; insbesondere von 30 bis 80 %; insbesondere von 40 bis 70 % der R₃-Gruppen in dem Polymer von Formel (I) für einen Teil mit einem zwitterionischen Polymer von Formel (II), wie in einem der vorhergehenden Ansprüche definiert, steht.

4. Das Polymer von einem der vorhergehenden Ansprüche, wobei r für 1 steht und der Teil mit einem zwitterionischen Polymer von Formel (II) einer ist, worin R'₄ für ein Aryl, Alkyl oder -S-R₅ steht, wobei R₅ wie oben definiert ist; insbesondere steht R'₄ für ein Aryl, Alkyl, -S-Aryl oder -S-Alkyl.

5. Das Polymer von einem der vorhergehenden Ansprüche, wobei der Teil mit einem zwitterionischen Polymer von Formel (II) einer ist, worin R₉ für -Alkylen-C(O)-O-alkylen-steht, wobei das Alkylen wahlweise substituiert ist.

6. Das Polymer von einem der vorhergehenden Ansprüche, wobei die verbleibenden R₃-Gruppen, die von einem Teil der Formel (II) verschieden sind, gleich oder verschieden sind und ausgewählt aus -((CH₂)ₚOH, -(CH₂)₂(OCH₂CH₂)_{q}OH, -(CH₂)ₚNH₂, und -(CH₂)₂(OCH₂CH₂)_{q}NH₂, sind, wobei p und q wie oben definiert sind.

7. Das Polymer von dem vorhergehenden Anspruch, wobei das oder jedes Oligopeptid der Formel (III) entspricht: 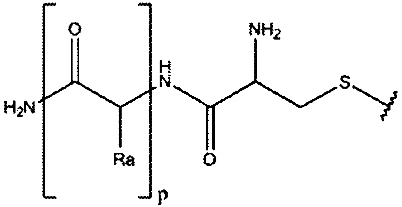 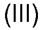 wobei p eine ganze Zahl von 2 bis 19 ist und wobei Ra ausgewählt ist aus der Gruppe bestehend aus H₂NC(=NH)-NH(CH₂)₃-, H₂N(CH₂)₄- oder (1H-Imidazol-4-yl)-CH₂-.

8. Das Polymer von einer der vorhergehenden Ansprüchen, das mit einem oder mehreren Zell-Targeting-Teilen funktionalisiert ist.

9. Ein Nanopartikel, umfassend ein oder mehrere Polymere der Formel (I) wie in einem der vorhergehenden Ansprüche definiert.

10. Das Nanopartikel von Anspruch 9, das ferner ein oder mehrere Polymere der Formel (Iter) umfasst: 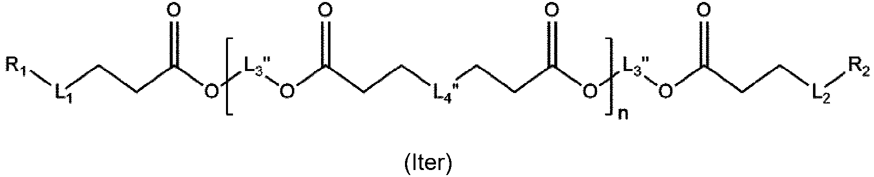
L"₃ ist unabhängig ausgewählt aus der Gruppe bestehend aus Alkylen, Alkenylen, Heteroalkylen, Heteroalkenylen, Arylen und Heteroarylen; oder, ersatzweise,
mindestens eines von L₃" ist
wobei
T₁" ist 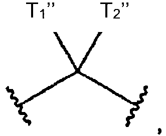 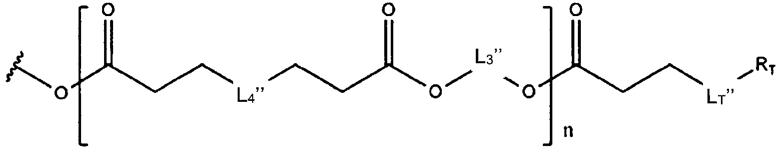
und T₂ ausgewählt ist aus H, Alkyl und 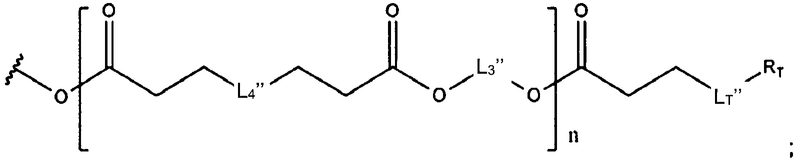
wobei L_{T}" unabhängig voneinander ausgewählt ist aus der Gruppe bestehend aus: 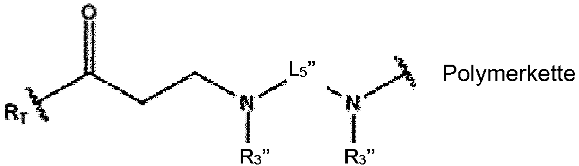
O, S, NRₓ und einer Bindung, wobei Rₓ unabhängig voneinander ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl, und
die verbleibenden L₃"-Gruppen in jedem Fall unabhängig voneinander ausgewählt sind aus der Gruppe bestehend aus Alkylen, Alkenylen, Heteroalkylen, Heteroalkenylen, Arylen und Heteroarylen;
L"₄ unabhängig voneinander ausgewählt ist aus der Gruppe bestehend aus (v) und (vi) 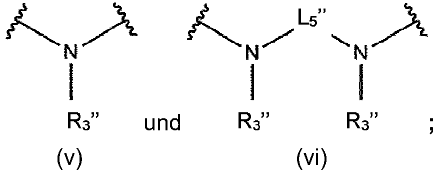
L₅" unabhängig voneinander ausgewählt ist aus der Gruppe bestehend aus Alkylen, Alkenylen, Heteroalkylen, Heteroalkenylen, Arylen und Heteroarylen;
jedes R₃" unabhängig voneinander ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl, Heteroaryl, Polyalkylenglycolen, einem Teil mit einer Hydroxylgruppe (-OH) und einem Teil mit einer Amingruppe (-NH₂),
wobei:
- das Polyalkylenglykol entweder direkt an das Stickstoffatom gebunden ist, an welches R₃" gebunden ist, oder über einen linkenden Teil an das Stickstoffatom gebunden ist, an welches R₃" gebunden ist, wobei der linkende Teil eine Alkylen-, Cycloalkylen-, Alkenylen-, Cycloalkenylen-, Heteroalkylen-, Heterocycloalkylen-, Arylen- oder Heteroarylengruppe ist;
- der Teil mit einer Hydroxylgruppe (-OH) ausgewählt ist aus der Gruppe bestehend aus - (CH₂)ₚOH und -(CH₂)₂(OCH₂CH₂)_{q}OH; und
- der Teil mit einer Aminogruppe (-NH₂) ausgewählt ist aus der Gruppe bestehend aus -(CH₂)ₚNH₂, -(CH₂)ₚOH;
n eine ganze Zahl von 5 bis 1000 ist, p eine ganze Zahl von 1 bis 20 ist und q eine ganze Zahl von 1 bis 10 ist,
jedes L₁ und L₂ unabhängig voneinander ausgewählt ist aus der Gruppe bestehend aus: 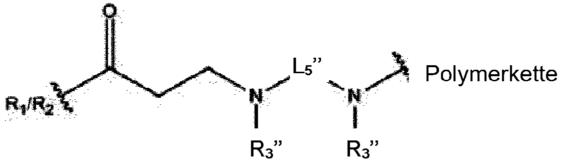
O, S, NRₓ und einer Bindung; wobei Rₓ unabhängig ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl; und R₃" und L₅" wie oben definiert sind; und
R₁, R₂ und R_{T} unabhängig voneinander aus einem Oligopeptid und R_{y} ausgewählt sind,
R_{y} ausgewählt ist aus der Gruppe bestehend aus Wasserstoff, Halogen, Alkyl, Cycloalkyl, Alkenyl, Cycloalkenyl, Heteroalkyl, Heterocycloalkyl, Acyl, Aryl und Heteroaryl; und unter der Voraussetzung, dass mindestens eines von R₁, R₂ und R_{T} ein Oligopeptid ist.

11. Das Nanopartikel von einem der vorhergehenden Ansprüche 9 bis 10, das eine oder mehrere Verbindungen von Interesse umfasst, insbesondere ausgewählt aus einem diagnostischen Reagenz, einem bildgebenden Reagenz und einem Wirkstoff.

12. Eine pharmazeutische Zusammensetzung umfassend eine therapeutisch wirksame Menge des Nanopartikels wie in einem der Ansprüche 9 bis 11 definiert, zusammen mit einem oder mehreren pharmazeutisch akzeptablen Hilfsstoffen.

13. Ein Abgabesystem umfassend eine äußere Beschichtung umfassend das Polymer (I) wie in einem der Ansprüche 1 bis 8 definiert und, wahlweise, ein oder mehrere Polymere der Formel (Iter) wie in Anspruch 10 definiert.

14. Verwendung des Polymers der Formel (I), wie in einem der Ansprüche 1 bis 8 definiert, allein oder in Kombination mit einem oder mehreren Polymeren der Formel (Iter), wie in Anspruch 10 definiert, als Beschichtung, insbesondere als Antifouling-Beschichtung; oder ersatzweise als Verkapselungsmittel; oder ersatzweise als Abagabeträger.

15. Das Nanopartikel wie in einem der Ansprüche 9 bis 11 definiert oder die pharmazeutische Zusammensetzung wie in Anspruch 12 definiert oder das Abgabesystem wie in Anspruch 13 definiert zur Verwendung in der Therapie.

## Revendications

1. Un polymère poly(bêta-aminoester) de formule (I) ou un sel pharmaceutiquement acceptable de celui-ci : 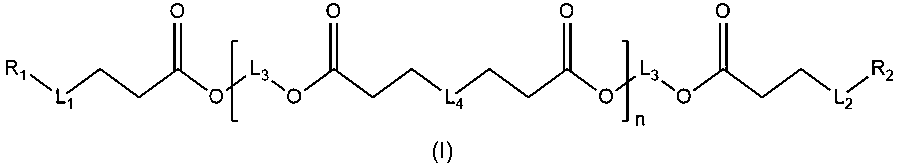 où
L₃ est indépendamment choisi dans le groupe constitué par alkylène, alcénylène, hétéroalkylène, hétéroalkénylène, arylène et hétéroarylène ; ou, en variante,
au moins l'un de L₃ est
où T₁ est 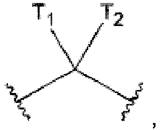 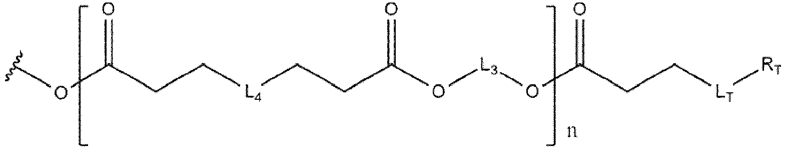
et T₂ est choisi parmi H, alkyle, et 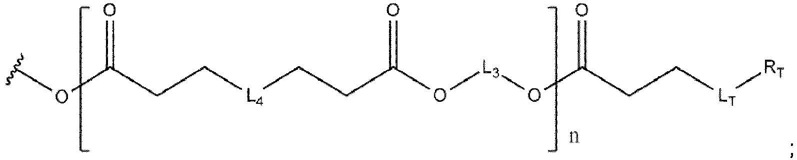
où L_{T} est indépendamment choisi dans le groupe constitué par : 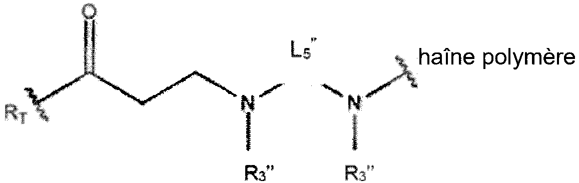
O, S, NRₓ et une liaison, dans laquelle Rₓ est indépendamment choisi dans le groupe constitué par hydrogène, halogène, alkyle, cycloalkyle, alcényle, cycloalcényle, hétéroalkyle, hétérocycloalkyle, acyle, aryle et hétéroaryle,
et les groupes L₃ restants sont indépendamment choisis dans le groupe constitué par alkylène, alcénylène, hétéroalkylène, hétéroalkénylène, arylène et hétéroarylène ;
L₄ est indépendamment choisi dans le groupe constitué par les formules (i) et (ii) 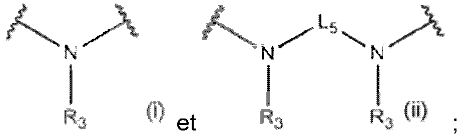
L₅ est indépendamment choisi dans le groupe constitué par alkylène, alcénylène, hétéroalkylène, hétéroalkénylène, arylène et hétéroarylène ;
chaque R₃ est indépendamment choisi dans le groupe constitué par hydrogène, halogène, alkyle, cycloalkyle, alcényle, cycloalcényle, hétéroalkyle, hétérocycloalkyle, acyle, aryle, hétéroaryle, polyalkylène glycols, une fraction ayant un groupe hydroxyle (-OH), une fraction ayant un groupe amine (-NH₂) et une fraction ayant un polymère zwitterionique,
dans lequel :
- ledit polyalkylène glycol est soit lié directement à l'atome d'azote auquel R₃ est attaché, soit lié à l'atome d'azote auquel R₃ est attaché via une fraction de liaison, ladite fraction de liaison est un groupe alkylène, cycloalkylène, alcénylène, cycloalkénylène, hétéroalkylène, hétérocycloalkylène, arylène ou hétéroarylène ;
- la fraction ayant un groupe hydroxyle (-OH) est choisie dans le groupe constitué par -(CH₂)ₚOH et -(CH₂)₂(OCH₂CH₂)_{q}OH ;
- la fraction ayant un groupe amino (-NH₂) est choisie dans le groupe constitué par -(CH₂)ₚNH₂ et -(CH₂)₂(OCH₂CH₂)_{q}NH₂ ; et
- la fraction ayant un polymère zwitterionique est l'une de formule (II) 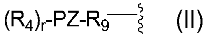
dans laquelle R₄ représente R'₄-C(=S)-S- ; R'₄ représente un aryle, hétéroaryle, alkyle, -SR₅, -NR₆R₇ ou -OR₈; R₅ représente aryle, hétéroaryle ou alkyle ; R₆ et R₇ sont identiques ou différents et représentent hydrogène, halogène, alkyle, cycloalkyle, alcényle, cycloalcényle, hétéroalkyle, hétérocycloalkyle, acyle, aryle et hétéroaryle ; R₅ représente hydrogène, alkyle, cycloalkyle, alcényle, cycloalcényle, hétéroalkyle, hétérocycloalkyle, acyle, aryle et hétéroaryle ; et R₉ est un groupe de liaison qui lie le PZ au -N- du substituant de formule (i) ou (ii) ;
PZ représente un polymère zwitterionique comprenant un ou plusieurs monomères zwitterioniques ;
à condition qu'au moins l'un des R₃ soit une fraction ayant un polymère zwitterionique tel que défini ci-dessus ;
n est un nombre entier de 5 à 1000, p est un nombre entier de 1 à 20, q est un nombre entier de 1 à 10, et r est un nombre entier de 0 à 1 ;
chaque L₁ et L₂ est indépendamment choisi dans le groupe constitué par : 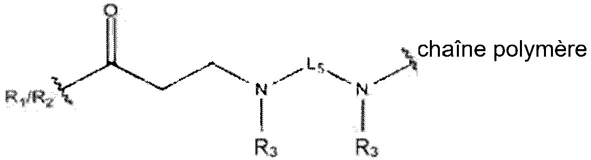
O, S, NRₓ et une liaison ; où Rₓ est indépendamment choisi dans le groupe constitué par hydrogène, halogène, alkyle, cycloalkyle, alcényle, cycloalcényle, hétéroalkyle, hétérocycloalkyle, acyle, aryle et hétéroaryle ; et R₃ et L₅ étant tels que définis ci-dessus ; et
R₁, R₂ et R_{T} sont indépendamment choisis parmi un oligopeptide et R_{y},
R_{y} étant choisi dans le groupe constitué par hydrogène, halogène, alkyle, cycloalkyle, alcényle, cycloalcényle, hétéroalkyle, hétérocycloalkyle, acyle, aryle et hétéroaryle ; et
à condition qu'au moins l'un de R₁, R₂ et R_{T} soit un oligopeptide.

2. Le polymère de la revendication 1, dans lequel :
L₁ et L₂ représentent une liaison ;
L₃ est indépendamment choisi dans le groupe constitué par alkylène, alcénylène, hétéroalkylène, hétéroalkénylène, arylène et hétéroarylène ; en particulier L₃ représente alkylène ; et
L₄ représente 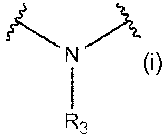
où R₃ est tel que défini dans la revendication 1.

3. Le polymère de l'une quelconque des revendications précédentes, dans lequel au moins 5 % des groupes R₃ dans le polymère de formule (I) représentent une fraction ayant un polymère zwitterionique de formule (II) tel que défini dans l'une quelconque des revendications ci-dessus ; en particulier au moins un 30 %, 40 %, 50 %, 60 %, 70 %, 70 %, 80 % ou 90 % des groupes R₃ dans le polymère de formule (I) représentent une fraction ayant un polymère zwitterionique de formule (II) tel que défini dans l'une quelconque des revendications ci-dessus ; en particulier au moins un 40 % des groupes R₃ dans le polymère de formule (I) représentent une fraction ayant un polymère zwitterionique de formule (II) tel que défini dans l'une quelconque des revendications ci-dessus ; en particulier de 30 à 80 % ; en particulier de 40 à 70 % des groupes R₃ dans le polymère de formule (I) représentent une fraction ayant un polymère zwitterionique de formule (II) tel que défini dans l'une quelconque des revendications ci-dessus.

4. Le polymère de l'une quelconque des revendications précédentes, dans lequel r représente 1 et la fraction ayant un polymère zwitterionique de formule (II) en est une où R'₄ représente un aryle, alkyle ou -S-R₅, où R₅ est tel que défini ci-dessus ; en particulier, R'₄ représente un aryle, alkyle, -S-aryle ou -S-alkyle.

5. Le polymère de l'une quelconque des revendications précédentes, dans lequel la fraction ayant un polymère zwitterionique de formule (II) en est une où R₉ représente-alkylène-C(O)-O-alkylène-, où l'alkylène est facultativement substitué.

6. Le polymère de l'une quelconque des revendications précédentes, dans lequel les groupes R₃ restants, qui sont autres qu'une fraction de formule (II), sont identiques ou différents et sont choisis parmi -(CH₂)ₚOH, -(CH₂)₂(OCH₂CH₂)_{q}OH, -(CH₂)ₚNH₂ et -(CH₂)₂(OCH₂CH₂)_{q}NH₂, où p et q sont tels que définis ci-dessus.

7. Le polymère de la revendication précédente, dans lequel le ou chaque oligopeptide est de formule (III) : 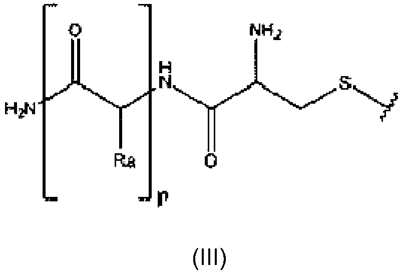 dans laquelle p est un nombre entier allant de 2 à 19 et dans laquelle Ra est choisi dans le groupe constitué par H₂NC(=NH)-NH(CH₂)₃-, H₂N(CH₂)₄- ou (1H-imidazol-4-yl)-CH₂-.

8. Le polymère de l'une quelconque des revendications précédentes, qui est fonctionnalisé avec une ou plusieurs fractions ciblant les cellules.

9. Une nanoparticule, comprenant un ou plusieurs polymères de formule (I) tels que définis dans l'une quelconque des revendications précédentes.

10. La nanoparticule de la revendication 9, qui comprend en outre un ou plusieurs polymères de formule (Iter) : 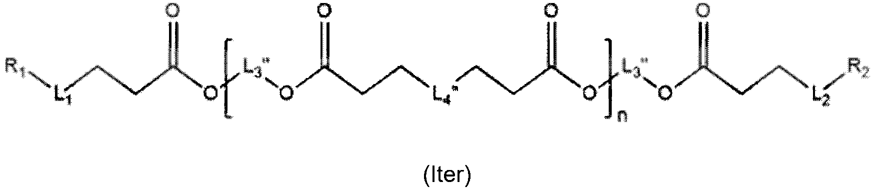
L"₃ est indépendamment choisi dans le groupe constitué par alkylène, alcénylène, hétéroalkylène, hétéroalkénylène, arylène et hétéroarylène ; ou, en variante,
au moins l'un des L₃" est
où
T₁" est 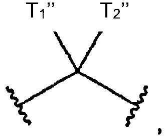 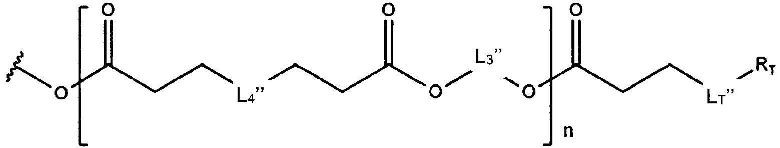
et T₂" est choisi parmi H, alkyle, et 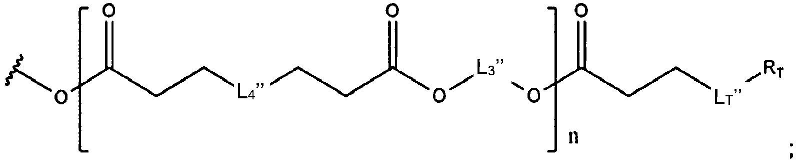
où L_{T}" est indépendamment choisi dans le groupe constitué par : 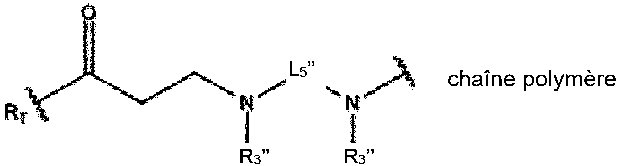
O, S, NRₓ et une liaison, où Rₓ est indépendamment choisi dans le groupe constitué par hydrogène, halogène, alkyle, cycloalkyle, alcényle, cycloalcényle, hétéroalkyle, hétérocycloalkyle, acyle, aryle et hétéroaryle, et
les groupes L₃" restants sont choisis indépendamment dans chaque cas dans le groupe constitué par alkylène, alcénylène, hétéroalkylène, hétéroalcénylène, arylène et hétéroarylène ;
L"₄ est indépendamment choisi dans le groupe constitué par (v) et (vi) 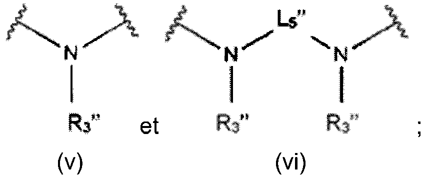
L₅" est indépendamment choisi dans le groupe constitué par alkylène, alcénylène, hétéroalkylène, hétéroalkénylène, arylène et hétéroarylène ;
chaque R₃" est indépendamment choisi dans le groupe constitué par hydrogène, halogène, alkyle, cycloalkyle, alcényle, cycloalcényle, hétéroalkyle, hétérocycloalkyle, acyle, aryle, hétéroaryle, polyalkylène glycols, une fraction ayant un groupe hydroxyle (-OH) et une fraction ayant un groupe amine (-NH₂),
dans laquelle :
- ledit polyalkylène glycol est soit lié directement à l'atome d'azote auquel R₃" est attaché, soit lié à l'atome d'azote auquel R₃" est attaché via une fraction de liaison, dans laquelle ladite fraction de liaison est un groupe alkylène, cycloalkylène, alcénylène, cycloalkénylène, hétéroalkylène, hétérocycloalkylène, arylène ou hétéroarylène ;
- la fraction ayant un groupe hydroxyle (-OH) est choisie dans le groupe constitué par - (CH₂)ₚOH et -(CH₂)₂(OCH₂CH₂)_{q}OH ; et
- la fraction ayant un groupe amino (-NH₂) est choisie dans le groupe constitué par -(CH₂)ₚNH₂, -(CH₂)ₚOH ;
n est un nombre entier de 5 à 1000, p est un nombre entier de 1 à 20, et q est un nombre entier de 1 à 10,
chaque L₁ et L₂ est indépendamment choisi dans le groupe constitué par : 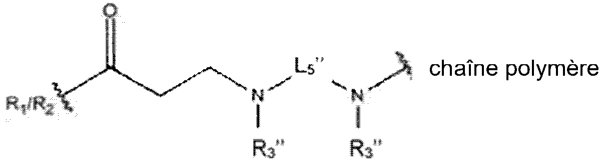
O, S, NRₓ et une liaison ; où Rₓ est indépendamment choisi dans le groupe constitué par hydrogène, halogène, alkyle, cycloalkyle, alcényle, cycloalcényle, hétéroalkyle, hétérocycloalkyle, acyle, aryle et hétéroaryle ; et R₃" et L₅" étant tels que définis ci-dessus ; et
R₁, R₂ et R_{T} sont indépendamment choisis parmi un oligopeptide et R_{y},
R_{y} étant choisi dans le groupe constitué par hydrogène, halogène, alkyle, cycloalkyle, alcényle, cycloalcényle, hétéroalkyle, hétérocycloalkyle, acyle, aryle et hétéroaryle ; et à condition qu'au moins l'un de R₁, R₂ et R_{T} soit un oligopeptide.

11. La nanoparticule de l'une quelconque des revendications précédentes 9-10, qui comprend un ou plusieurs composés d'intérêt, en particulier choisis parmi un réactif de diagnostic, un réactif d'imagerie et un ingrédient actif.

12. Une composition pharmaceutique comprenant une quantité thérapeutiquement efficace de la nanoparticule telle que définie dans l'une quelconque des revendications 9 à 11, avec un ou plusieurs excipients pharmaceutiquement acceptables.

13. Un système d'administration comprenant un revêtement externe comprenant le polymère (I) tel que défini dans l'une quelconque des revendications 1 à 8 et, facultativement, un ou plusieurs polymères de formule (Iter) tels que définis dans la revendication 10.

14. Utilisation du polymère de formule (I) tel que défini dans l'une quelconque des revendications 1 à 8, seul ou en combinaison avec un ou plusieurs polymères de formule (Iter) tels que définis dans la revendication 10, en tant que revêtement, en particulier en tant que revêtement antisalissure ; ou, en variante, en tant qu'agent d'encapsulation ; ou, en variante, en tant que véhicule d'administration.

15. La nanoparticule telle que définie dans l'une quelconque des revendications 9 à 11 ou la composition pharmaceutique telle que définie dans la revendication 12 ou le système d'administration tel que défini dans la revendication 13 pour une utilisation en thérapie.
